# EUROPÄISCHE PATENTSCHRIFT

(11) **EP 3 423 542 B1**
(45) Veröffentlichungstag und Bekanntmachung des Hinweises auf die Patenterteilung: **22.07.2020**
(21) Anmeldenummer: 17703027.7
(22) Anmeldetag: 06.02.2017
(51) Int. Cl.: C09K 11/06, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 03.03.2016 EP 16158460; 11.03.2016 EP 16159829
(43) Veröffentlichungstag der Anmeldung: 09.01.2019
(73) Patentinhaber: Merck Patent GmbH, 64293 Darmstadt (DE)
(72) Erfinder: PARHAM, Amir, Hossain, 60486 Frankfurt am Main (DE); EBERLE, Thomas, 76829 Landau (DE); JATSCH, Anja, 60489 Frankfurt am Main (DE); GROSSMANN, Tobias, 64297 Darmstadt (DE); KROEBER, Jonas, Valentin, 60311 Frankfurt (DE); MONTENEGRO, Elvira, 69469 Weinheim (DE); WERN, Caroline, 64293 Darmstadt (DE)
(86) Internationale Anmeldenummer: PCT/EP2017/000154
(87) Internationale Veröffentlichungsnummer: WO 2017/148564

(56) Entgegenhaltungen:
- WO-A1-2016/015810
- WO-A1-2017/012687
- WO-A1-2017/016632
- WO-A2-2015/194791
- US-A1- 2016 028 014

## Beschreibung

Die vorliegende Erfindung beschreibt Amine mit Dibenzofuran-, Dibenzothiophen- und Fluorengruppen insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik sind Amine mit Fluoren und Dibenzofurangruppen aus US 2014/0284578 bekannt. Auch Verbindungen mit Carbazolen sind aus EP 2421064 bekannt. US 2013/0234118 offenbart Amine mit kondensierten aromatischen Gruppen wie Pyrenen.

Aus WO 2016/015810 sind Dibenzofuran- und Dibenzothiophenverbindungen bekannt, die in 1-Position mit einer Heteroarylgruppe und in 8-Position mit einer Diaryl- bzw. -heteroarylaminogruppe substituiert sind. Dabei ist die Anwesenheit der Heteroarylgruppe in 1-Position des charakterisierende Merkmal.

Aus US 2016/028014, WO 2017/016632 und WO 2017/012687 sind Triarylaminderivate bekannt, in denen der Stickstoff mit einer 1-Dibenzofuran- bzw. 1-Dibenzothiophengruppe, einer Spirobifluorengruppe und einer weiteren aromatischen bzw. heteroaromatischen Gruppe substituiert sind.

Aus WO 2015/194791 sind Triarylaminderivate bekannt, in welchen das Stickstoffatom in der 1-Position eines Dibenzothiophenderivats gebunden ist, wobei das Dibenzothiophenderivat zwei ankondensierte Benzogruppen umfasst.

Generell besteht bei diesen Materialien für die Verwendung als Matrixmaterialien noch Verbesserungsbedarf, insbesondere in Bezug auf die Lebensdauer und Oxidationsempfindlichkeit, aber auch in Bezug auf die Effizienz und die Betriebsspannung der Vorrichtung.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß der folgenden Formel (1), 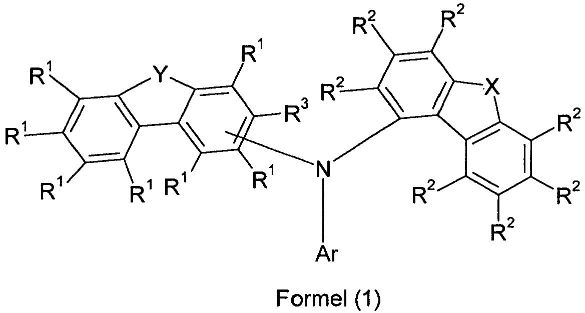 wobei für die verwendeten Symbole gilt:
- X: ist O oder S;
- Y: ist O, S oder CR₂;
- Ar: ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann, wobei Ar nur Arylgruppen oder Heteroarylgruppen mit bis zu 15 aromatischen Ringatomen und keine Carbazolylgruppen als Heteroarylgruppen, sowie keine 9,9'-Spirobifluoren-gruppen umfasst;
- R: ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxyoder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁴ substituiert sein kann;
- R¹: ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 CAtomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R¹ oder zwei Substituenten R¹ und R³, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
- R²: ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R², die an benachbarte Kohlenstoffatome gebunden sind, ein monocyclisches oder polycyclisches, aliphatisches Ringsystem bilden, das mit einem oder mehreren Resten R⁴ substituiert sein kann;
- R³: ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R³ und R¹, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
- Ar¹: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁴ substituiert sein kann; dabei können zwei Reste Ar¹, welche an dasselbe P-Atom oder B-Atom binden, auch durch eine Einfachbindung oder eine Brücke, ausgewählt aus N(R⁴), C(R⁴)₂, O oder S, miteinander verbrückt sein;
- R⁴: ist bei jedem Auftreten gleich oder verschieden ausgewählt aus der Gruppe bestehend aus H, D, F, Cl, Br, I, CN, NO₂, N(R⁵)₂, C(=O)R⁵, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei Substituenten R⁴, die an das gleiche Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R⁵ substituiert sein kann;
- R⁵: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein mono- oder polycyclisches, aliphatisches Ringsystem bilden;
wobei einmal an Stelle von R¹ oder R³ das Stickstoffatom an das entsprechende Kohlenstoffatom gebunden ist, wobei Y im Falle von R³ nicht für CR₂ steht;
Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht. 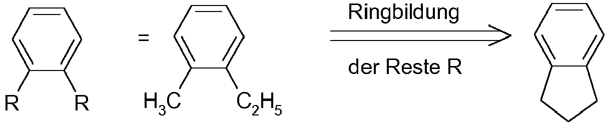 Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

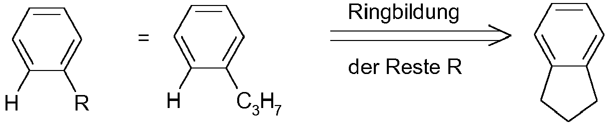

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt gilt für Formel (1), dass für Y gleich CR₂ statt R¹ das Stickstoffatom an das entsprechende Kohlenstoffatom gebunden ist; und dass für Y gleich O oder S statt R¹ oder R³ das Stickstoffatom an das entsprechende Kohlenstoffatom gebunden ist.

In einer bevorzugten Ausführungsform steht X für O.

In einer weiteren bevorzugten Ausführungsform ist Y gleich CR₂.

In einer weiteren bevorzugten Ausführungsform steht X für O und Y für CR2.

In einer weiteren bevorzugten Ausführungsform der Erfindung gilt, dass, wenn die Verbindung der Formel (1) einen Substituenten R¹ oder R² aufweist, der für ein gegebenenfalls substituiertes Carbazol steht, dann ist dieses Carbazol nicht über seine 2-Position gebunden.

In einer weiteren Ausführungsform der Erfindung ist die Verbindung ausgewählt aus Verbindungen der Formel (1-1) oder Formel (1-2), 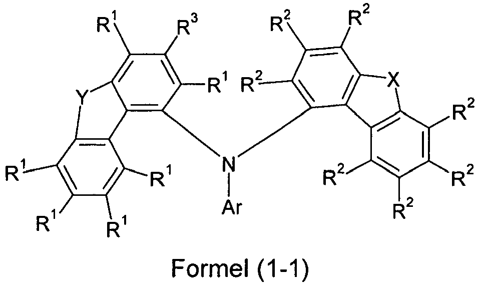 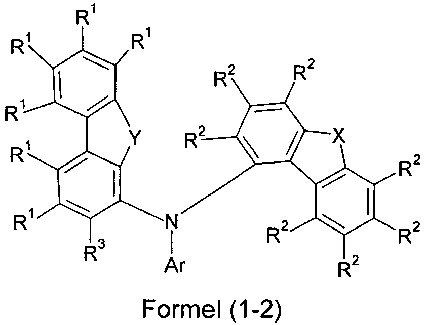 wobei die Symbole den Symbolen von Formel (1) entsprechen.

In einer weiteren bevorzugten Ausführungsform der Erfindung steht Ar gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R substituiert sein kann, bevorzugt aber unsubstituiert ist, wobei Ar nur Arylgruppen oder Heteroarylgruppen mit bis zu 15 aromatischen Ringatomen und keine Carbazolylgruppe und keine 9,9'-Spirobifluorengruppe umfasst. Beispiele für geeignete Gruppen Ar sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, Pyridyl, Pyrimidinyl, 1-, 2-, 3- oder 4-Dibenzofuranyl und 1-, 2-, 3- oder 4-Dibenzothienyl, die jeweils durch einen oder mehrere Reste R substituiert sein können, bevorzugt aber unsubstituiert sind.

In einer bevorzugten Ausführungsform der Erfindung ist Ar ausgewählt aus den Strukturen der Formeln (Ar-1) bis (Ar-16), 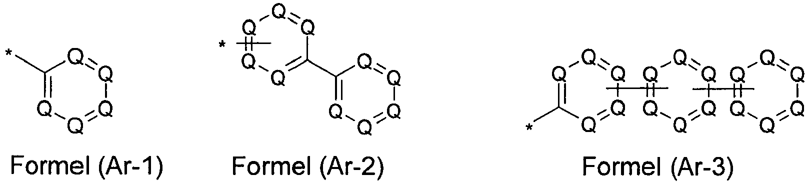 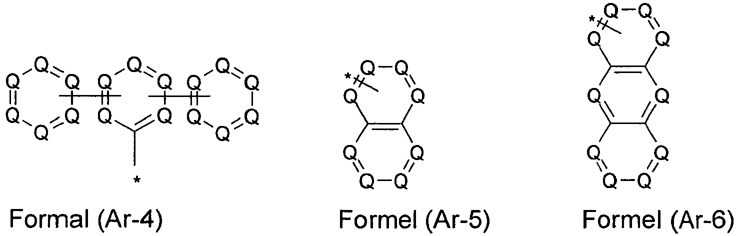 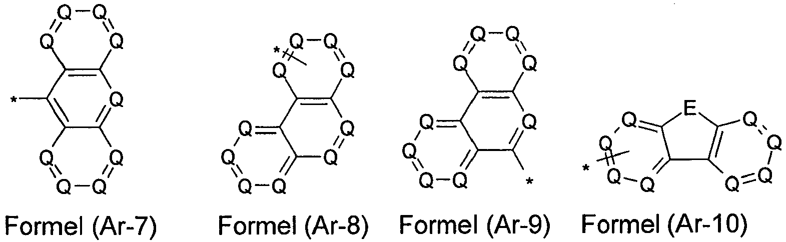 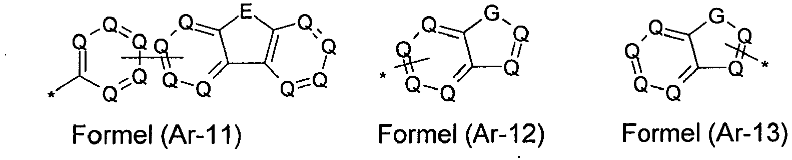 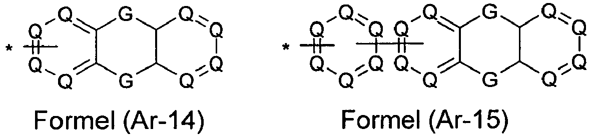 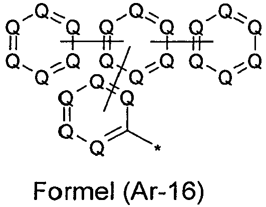 wobei die Symbole den Symbolen der Formel (1) entsprechen und zusätzlich gilt:
- Q: ist bei jedem Auftreten gleich oder verschieden CR⁴ oder N, wobei maximal 3 Symbole Q pro Cyclus für N stehen;
- E: ist bei jedem Auftreten gleich oder verschieden C(R⁴)₂, O, S oder C=O, wobei beide R⁴ kein aromatisches oder heteroaromatisches Ringsystem bilden;
- G: ist bei jedem Auftreten NR⁴, C(R⁴)₂, O, S oder C=O; und
- *: die Bindung zum Stickstoffatom darstellt.

### Bevorzugt steht kein Q für N.

In einer weiteren bevorzugten Ausführungsform ist die Gruppe Ar bei jedem Auftreten ausgewählt aus den Gruppen mit den Strukturen der Formeln (Ar-1) bis (Ar-16), wobei die allgemeinen Formeln durch die besonders bevorzugten Ausführungsformen jeweils gemäß der folgenden Formeln (Ar-1-1) bis (Ar-16-6) ersetzt werden (z. B. wird Formel (Ar-1) durch eine der Formeln (Ar-1-1) bis (Ar-1-9) ersetzt): 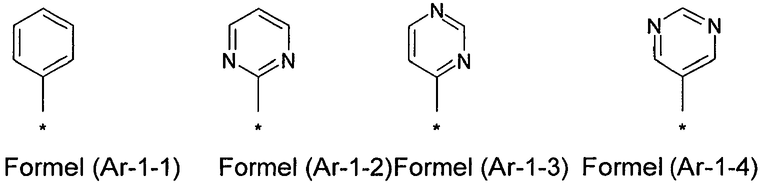 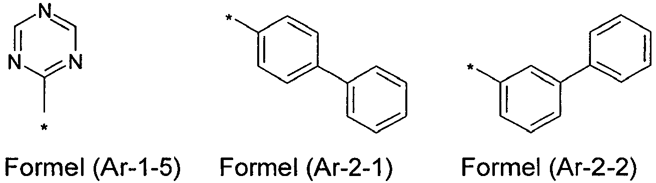 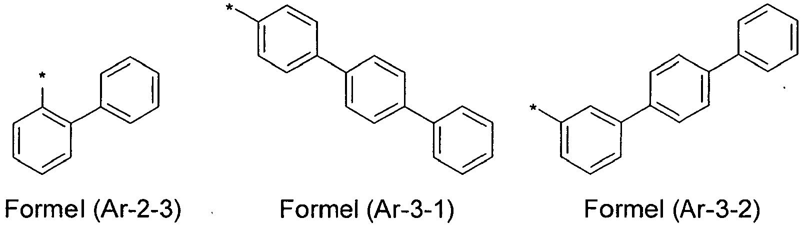 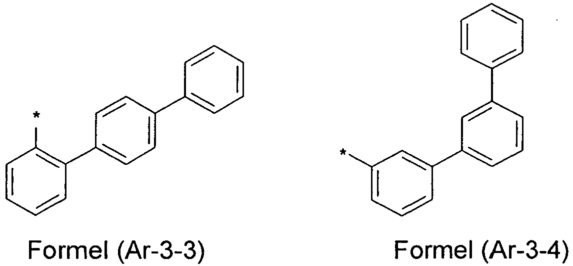 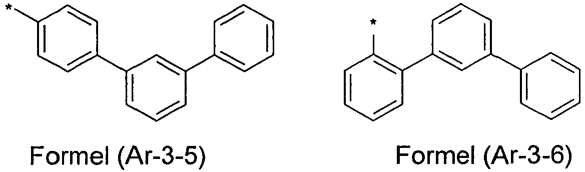 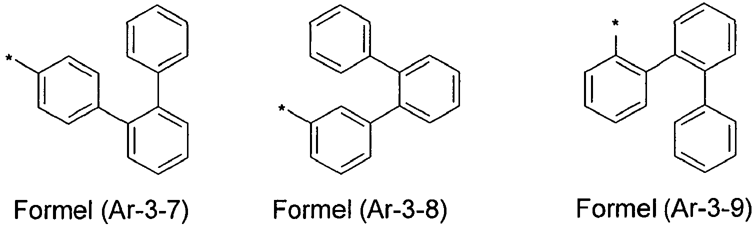 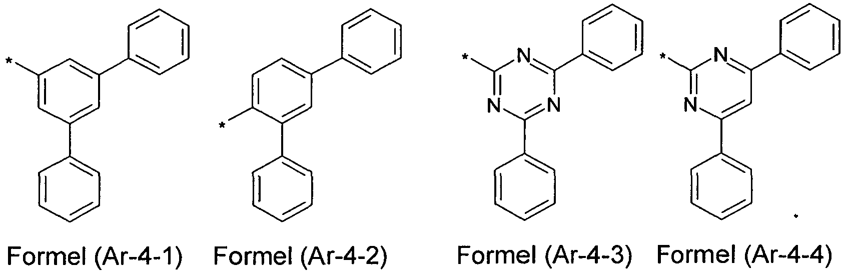 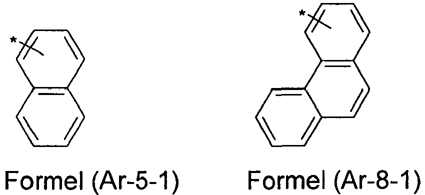 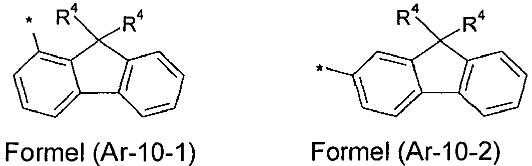 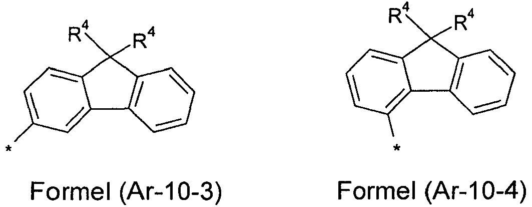 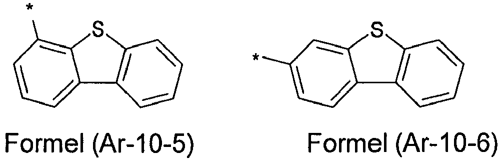 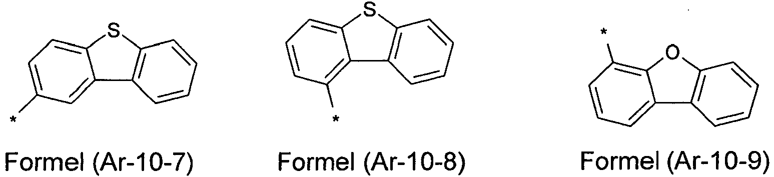 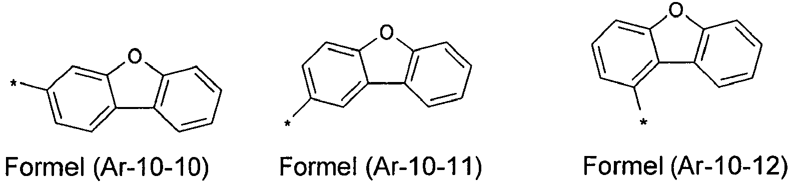 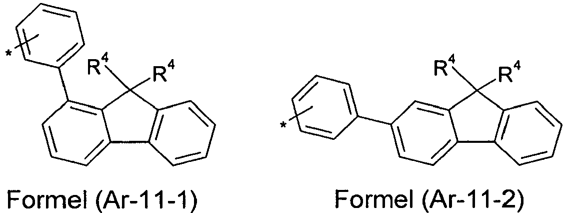 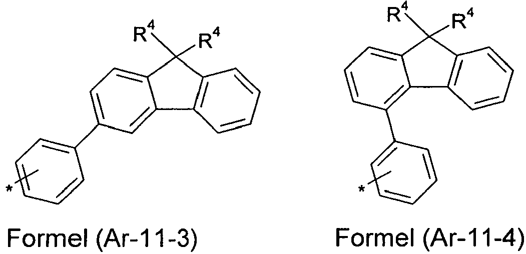 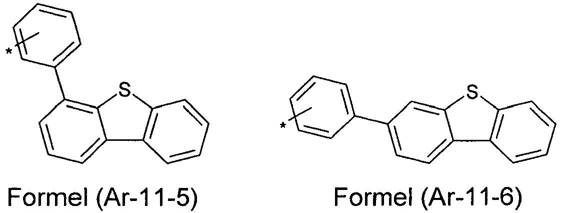 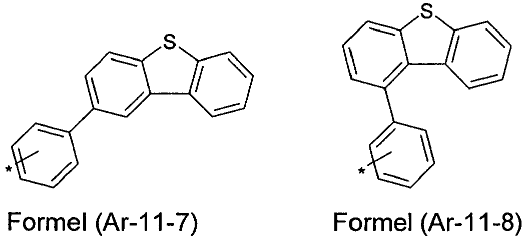 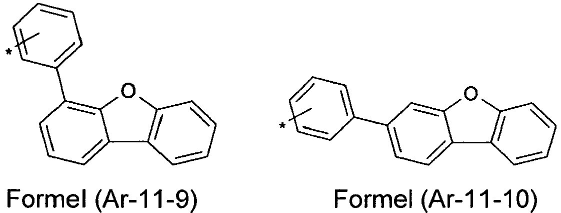 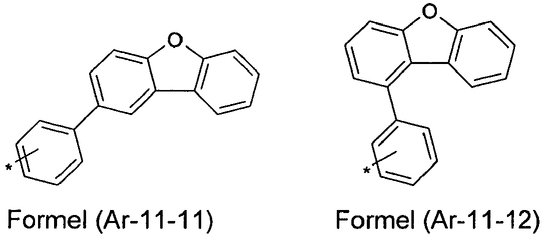 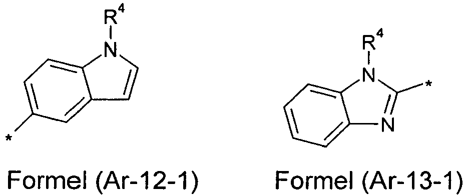 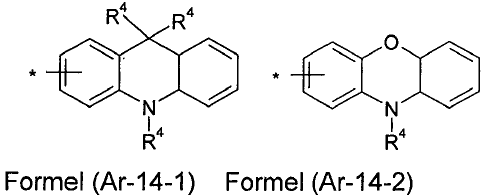 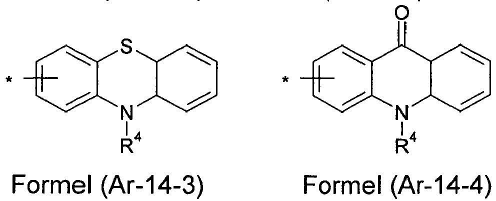 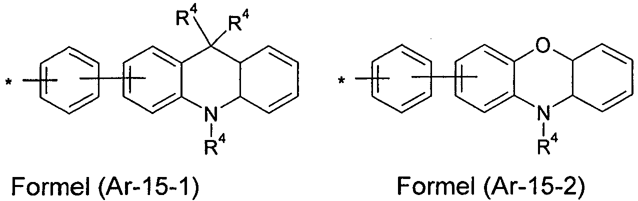 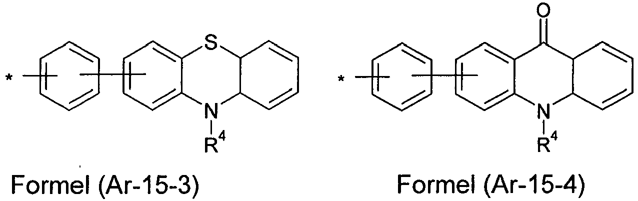 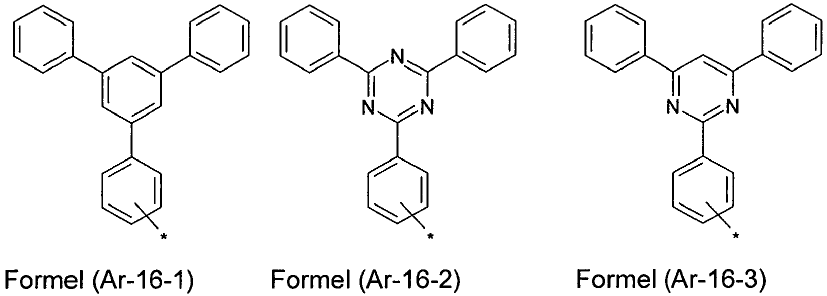 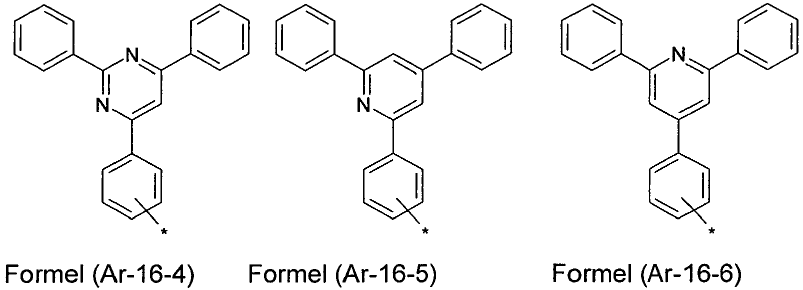 wobei die Symbole den Symbolen in Formel (Ar-1) bis (Ar-16) entsprechen. Die Formeln können an den freien Positionen mit R⁴ substituiert sein.

In einer weiteren Ausführungsform der Erfindung ist R² bei jedem Auftreten gleich oder verschieden im Falle eines aromatischen oder heteroaromatischen Ringsystems ausgewählt aus den Strukturen der Formeln (Ar-1) bis (Ar-16), bzw. ihren bevorzugten Ausführungsformen, und Strukturen der Formeln (Ar-17) und (Ar-18): 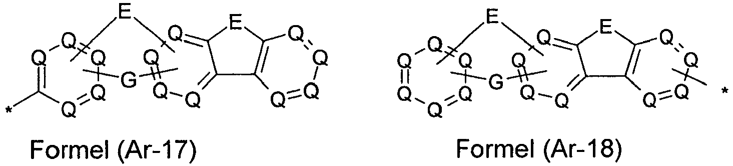 wobei die Symbole den Symbolen der Formel (1) entsprechen und zusätzlich für Formeln (Ar-17) und (Ar-18) gilt:
- Q: ist bei jedem Auftreten gleich oder verschieden CR⁴ oder N, wobei maximal 3 Symbole Q pro Cyclus für N stehen;
- E: ist bei jedem Auftreten gleich oder verschieden NR⁴, C(R⁴)₂, O, S oder C=O, wobei beide R⁴ kein aromatisches oder heteroaromatisches Ringsystem bilden;
- G: ist bei jedem Auftreten NR⁴, C(R⁴)₂, O, S oder C=O; und
- *: die Bindung zum aromatischen Ringsystem darstellt.

### Bevorzugt steht kein Q für N.

In einer weiteren bevorzugten Ausführungsform ist die Gruppe R² bei jedem Auftreten im Falle eines aromatischen oder heteroaromatischen Ringsystems ausgewählt aus den Gruppen mit den Strukturen der Formeln (Ar-1) bis (Ar-18), wobei die allgemeinen Formeln durch die besonders bevorzugten Ausführungsformen jeweils gemäß der folgenden Formeln (Ar-1-1) bis (Ar-16-6) ersetzt werden (z. B. wird Formel (Ar-1) durch eine der Formeln (Ar-1-1) bis (Ar-1-9) ersetzt), wobei zusätzlich folgende Formeln bevorzugt sind: 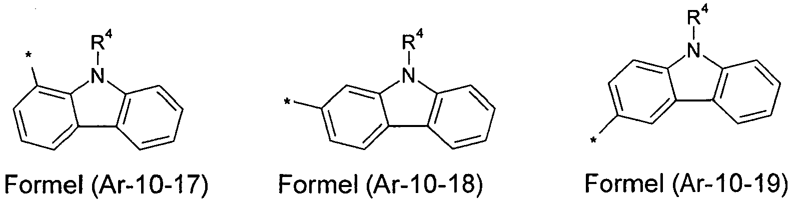 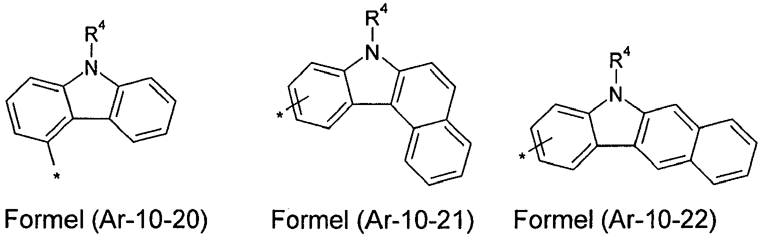 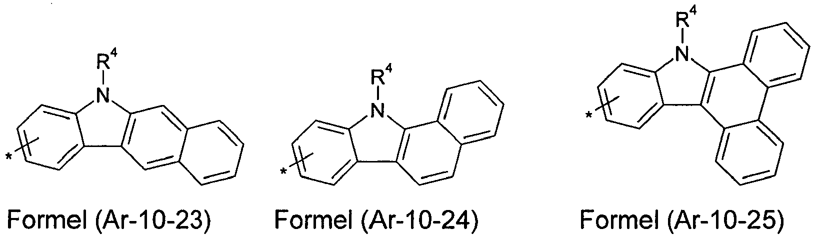 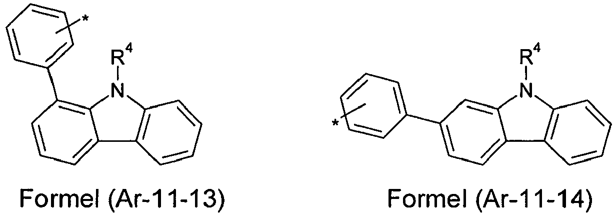 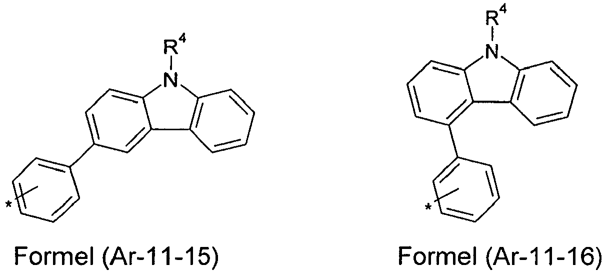 wobei die Symbole den Symbolen von Formel (1) entsprechen.

In einer Ausführungsform der Erfindung ist die Verbindung eine Struktur der Formel (2) oder Formel (3): 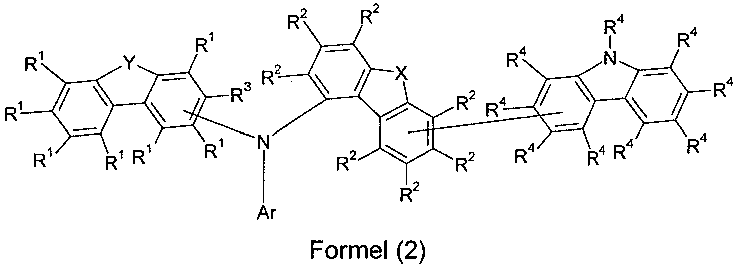 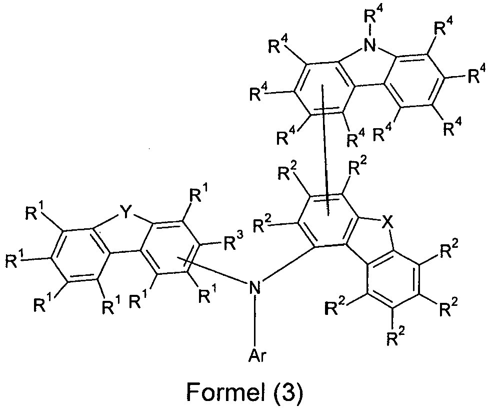 wobei die Symbole den Symbolen der Formel (1) entsprechen, wobei ein R² und ein an ein Kohlenstoffatom gebundenes R⁴ durch die Einfachbindung ersetzt sind, im Falle von R² bevorzugt am nicht mit dem Stickstoffatom verbundenen Sechsring gemäß Formel (2).

In einer bevorzugten Ausführungsform der Erfindung ist das Carbazol nicht über seine 2-Position gebunden.

In einer weiteren bevorzugten Ausführungsform der Erfindung ist die Verbindung eine Verbindung gemäß einer der Stukturen der Formeln (2-1) oder (2-2): 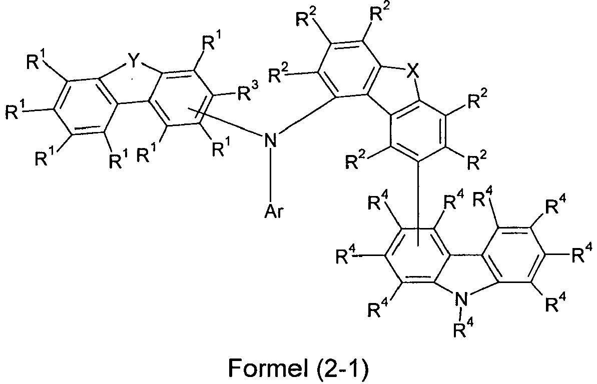 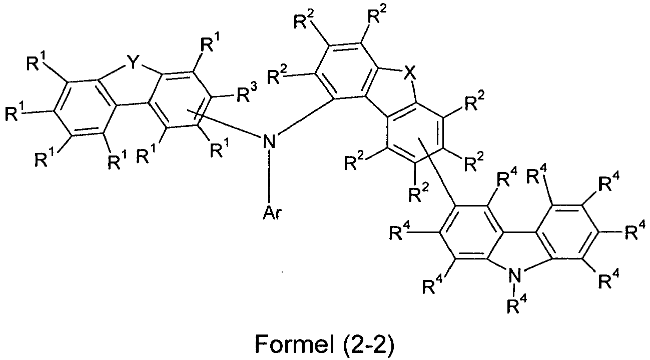 wobei die Symbole den Symbolen der Formel (2) entsprechen, wobei in Formel (2-1) ein R² und in Formel (2-2) ein an ein Kohlenstoffatom gebundenes R⁴ durch die Einfachbindung ersetzt sind, im Fall von Formel (2-2) am nicht mit dem Stickstoffatom verbundenen Sechsring.

In einer weiteren bevorzugten Ausführungsform der Erfindung ist die Verbindung eine Verbindung der Formel (2-3): 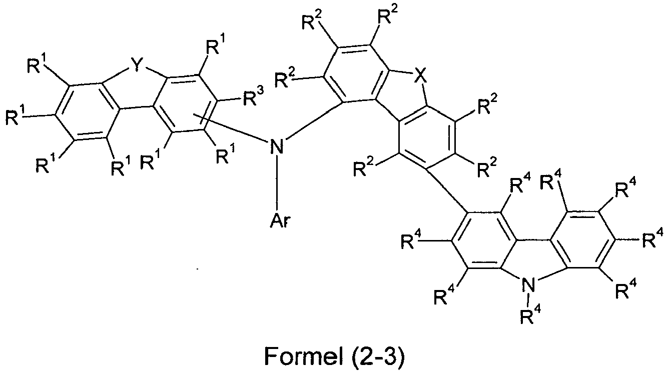 wobei die Symbole den Symbolen der Formel (2) entsprechen.

In einer weiteren bevorzugten Ausführungsform der Erfindung ist die Verbindung eine Verbindung gemäß einer der Stukturen der Formeln (3-1) oder (3-2): 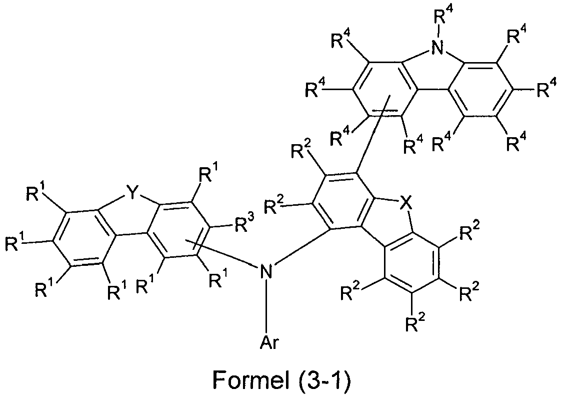 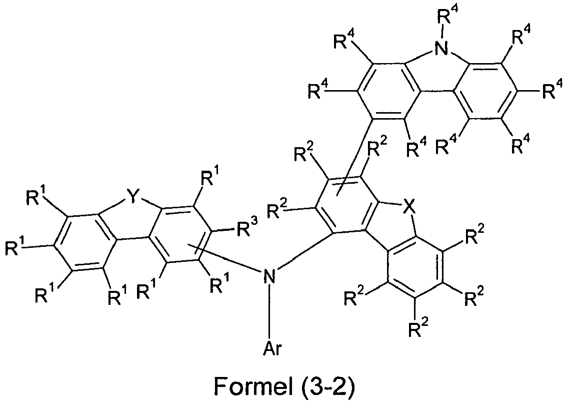 wobei die Symbole den Symbolen der Formel (3) entsprechen, wobei in Formel (3-1) ein R² und in Formel (3-2) ein an ein Kohlenstoffatom gebundenes R⁴ durch die Einfachbindung erstzt sind, im Fall von Formel (3-2) am mit dem Stickstoffatom verbundene Sechsring.

In einer weiteren bevorzugten Ausführungsform der Erfindung ist die Verbindung eine Verbindung der Formel (3-3): 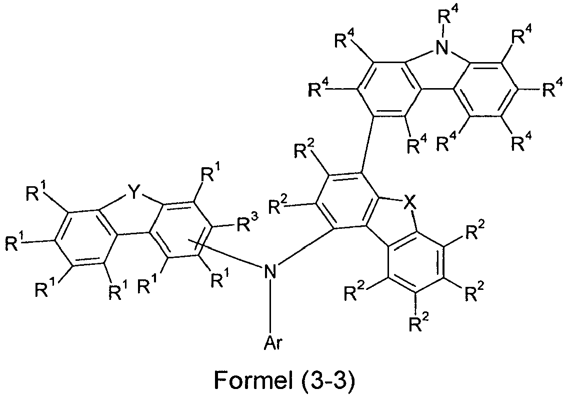 wobei die Symbole den Symbolen der Formel (3) entsprechen.

In einer weiteren Ausführungsform der Erfindung ist die Verbindung eine Verbindung der Formeln (2-1a), (2-1b), (2-2a), (2-2b): 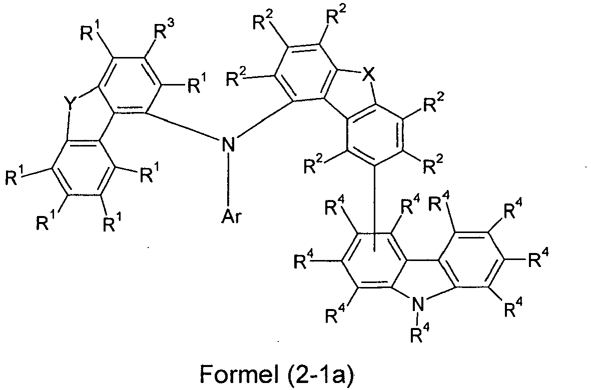 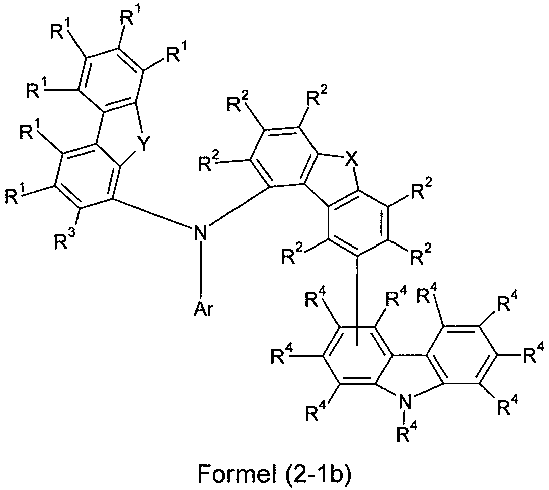 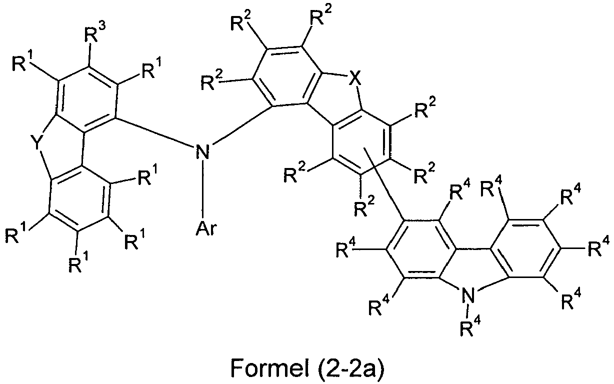 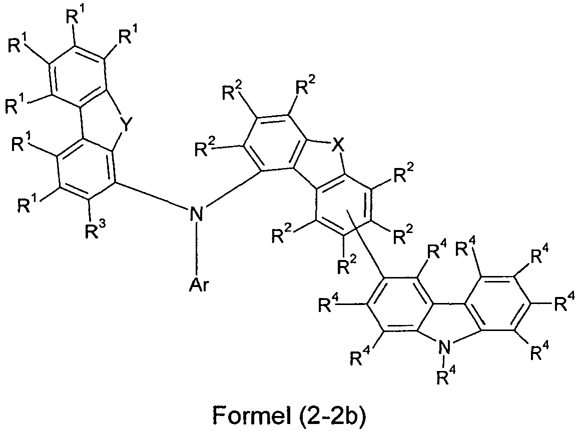 wobei die Symbole den Symbolen der Formel (2) entsprechen.

In einer weiteren Aufführungsform der Erfindung ist die Verbindung eine Verbindung der Formeln (3-1a), (3-1b), (3-2a) oder (3-2b): 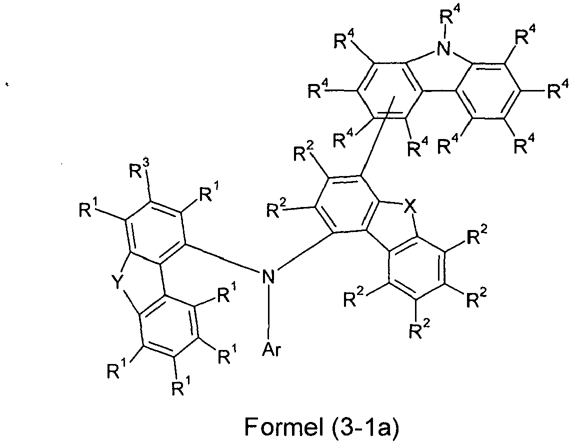 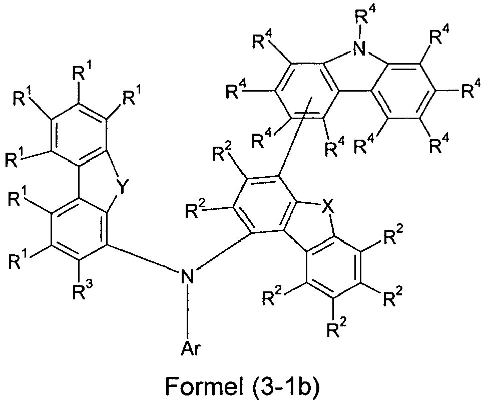 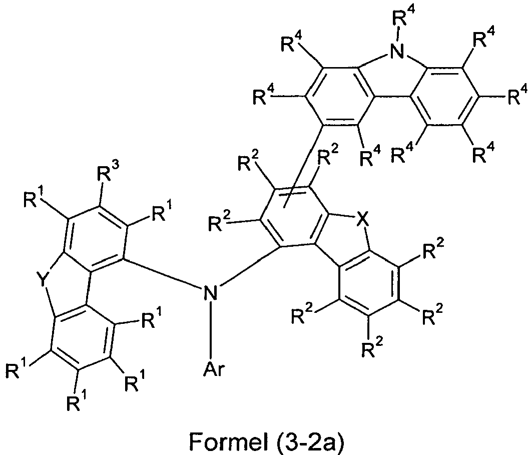 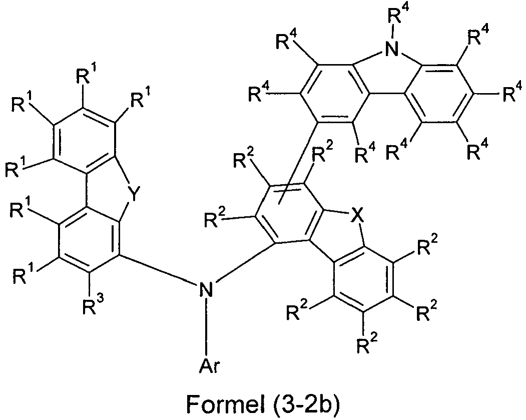 wobei die Symbole den Symbolen der Formel (3) entsprechen.

In einer weiteren bevorzugten Ausführungsform der Erfindung ist die Verbindung eine Verbindung der Formel (2-3a) oder (2-3b): 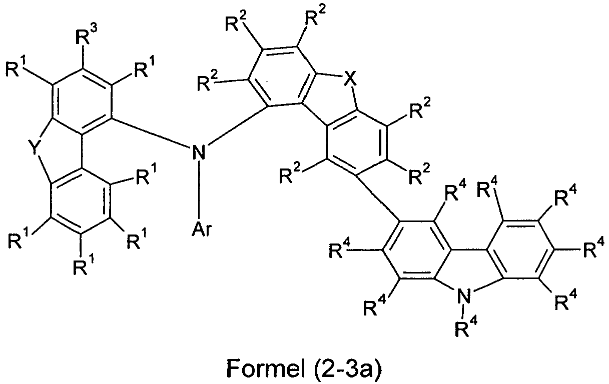 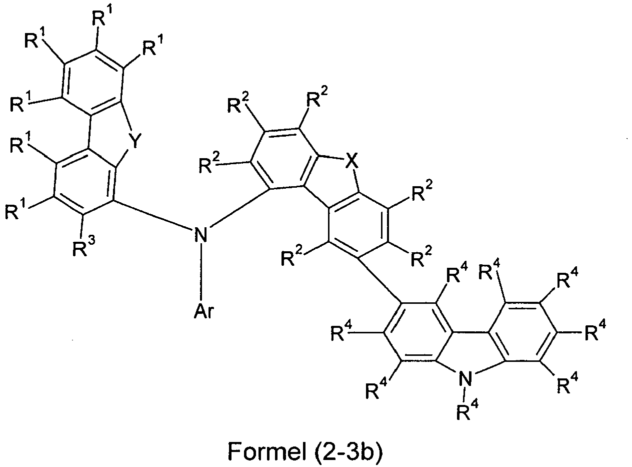 wobei die Symbole den Symbolen der Formel (2-3) entsprechen.

In einer weiteren bevorzugten Ausführungsform der Erfindung ist die Verbindung eine Verbindung der Formel (3-3a) oder (3-3b): 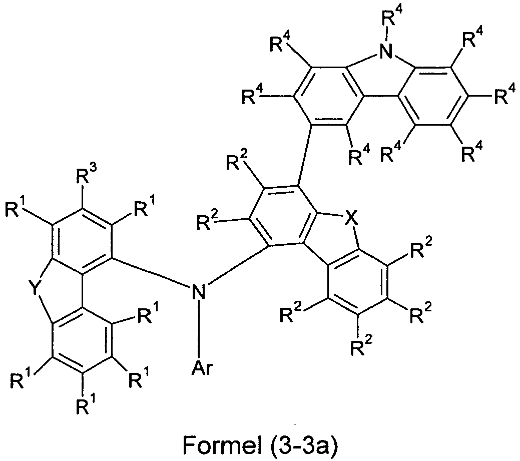 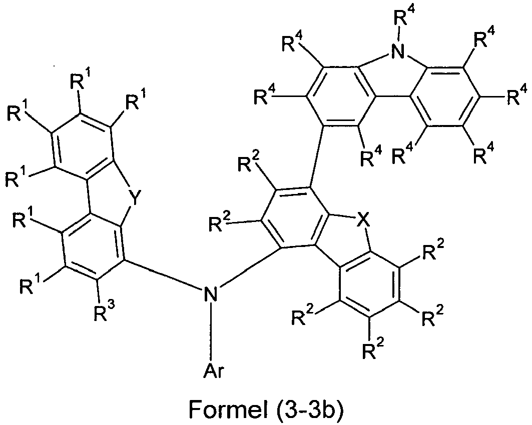 wobei die Symbole den Symbolen der Formel (3-3) entsprechen.

In einer weiteren Ausführungsform der Erfindung sind R¹ und R³ bei jedem Auftreten gleich oder verschieden im Falle eines aromatischen oder heteroaromatischen Ringsystems ausgewählt aus einer der Formeln (Ar-1) bis (Ar-16), wobei die Bedeutung der Symbole den Symbolen wie für Ar definiert entsprechen und gilt, dass * für die Bindung zum aromatischen Ringsystem steht.

Bevorzugt sind die Substituenten R, die an Ar gebunden sind, ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R, die an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Wenn Y für CR₂ steht, ist es bevorzugt, wenn die Reste R, die an dieses Kohlenstoffatom gebunden sind, bei jedem Auftreten gleich oder verschieden für eine geradkettige Alkylgruppe mit 1 bis 8 C-Atomen, bevorzugt mit 1, 2, 3 oder 4 C-Atomen, oder eine verzweigte oder cyclische Alkylgruppe mit 3 bis 8 C-Atomen, bevorzugt mit 3, 4, 5 oder 6 C-Atomen, oder eine Alkenylgruppe mit 2 bis 8 C-Atomen, bevorzugt mit 2, 3 oder 4 C-Atomen, stehen, wobei die Alkyl- bzw. Alkenylgruppe jeweils mit einem oder mehreren Resten R⁴ substituiert sein kann, wobei eine oder mehrere nicht-benachbarte CH₂-Gruppen durch O ersetzt sein können und wobei ein oder mehrere H-Atome durch D oder F ersetzt sein können, oder für ein aromatisches oder heteroaromatisches Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, stehen; dabei können optional die beiden Substituenten R ein monocyclisches oder polycyclisches, aliphatisches Ringsystem bilden, das mit einem oder mehreren Resten R⁴ substituiert sein kann. Durch Ringbildung der beiden Substituenten R wird ein Spirosystem aufgespannt.

Bevorzugt sind die Substituenten R¹ ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R⁴ substituiert sein kann, bevorzugt aber unsubstituiert ist, oder einem aromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R¹ oder zwei Substituenten R¹ und R³, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Bevorzugt sind die Substituenten R² ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R², die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Bevorzugt sind die Substituenten R³ ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R³ und R¹, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Bevorzugt sind die Substituenten R⁴ ausgewählt aus der Gruppe bestehend aus H, D, F, CN, N(Ar¹)₂, N(R⁵)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R⁵ substituiert sein kann, bevorzugt aber unsubstituiert ist; dabei können optional zwei Substituenten R⁴, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁵ substituiert sein kann, bevorzugt aber unsubstituiert ist.

Wenn E bzw. G für NR⁴ steht, ist es bevorzugt, wenn der Rest R⁴, der an dieses Stickstoffatom gebunden ist, bei jedem Auftreten gleich oder verschieden für ein aromatisches oder heteroaromatisches Ringsystem mit 5 bis 24 aromatischen Ringatomen steht, das jeweils mit einem oder mehreren Resten R⁵ substituiert sein kann, besonders bevorzugt für ein aromatisches oder heteroaromatisches Ringsystem mit 6 bis 18 aromatischen Ringatomen, das mit einem oder mehreren Resten R⁵ substituiert sein kann. Beispiele für geeignete Substituenten R⁴ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R⁵ ungleich H oder D substituiert ist. Dabei können diese Gruppen jeweils durch einen oder mehrere Reste R⁵ substituiert sein, sind bevorzugt aber unsubstituiert.

In einer weiteren Ausführungsform der Erfindung umfasst die Verbindung außer dem zentralen Stickstoffatom keine weiteren unverbrückten Amine. Dies bedeutet, dass zum Beispiel kein Rest für N(Ar¹)₂ oder N(R⁴)₂ steht.

In einer weiteren Ausführungsform der Erfindung steht mindestens ein R, R² oder R³ in Formel (1) für eine Heteroarylgruppe.

In einer weiteren Ausführungsform der Erfindung steht Ar nicht für eine Gruppe gemäß Formel (Ar-10-2) wie eine 2-Fluorenylgruppe.

Die oben genannten Bevorzugungen können einzeln oder gemeinsam auftreten. Es ist bevorzugt, wenn die oben genannten Bevorzugungen gemeinsam auftreten.

Beispiele für geeignete erfindungsgemäße Verbindungen sind die nachstehend gezeigten Strukturen.

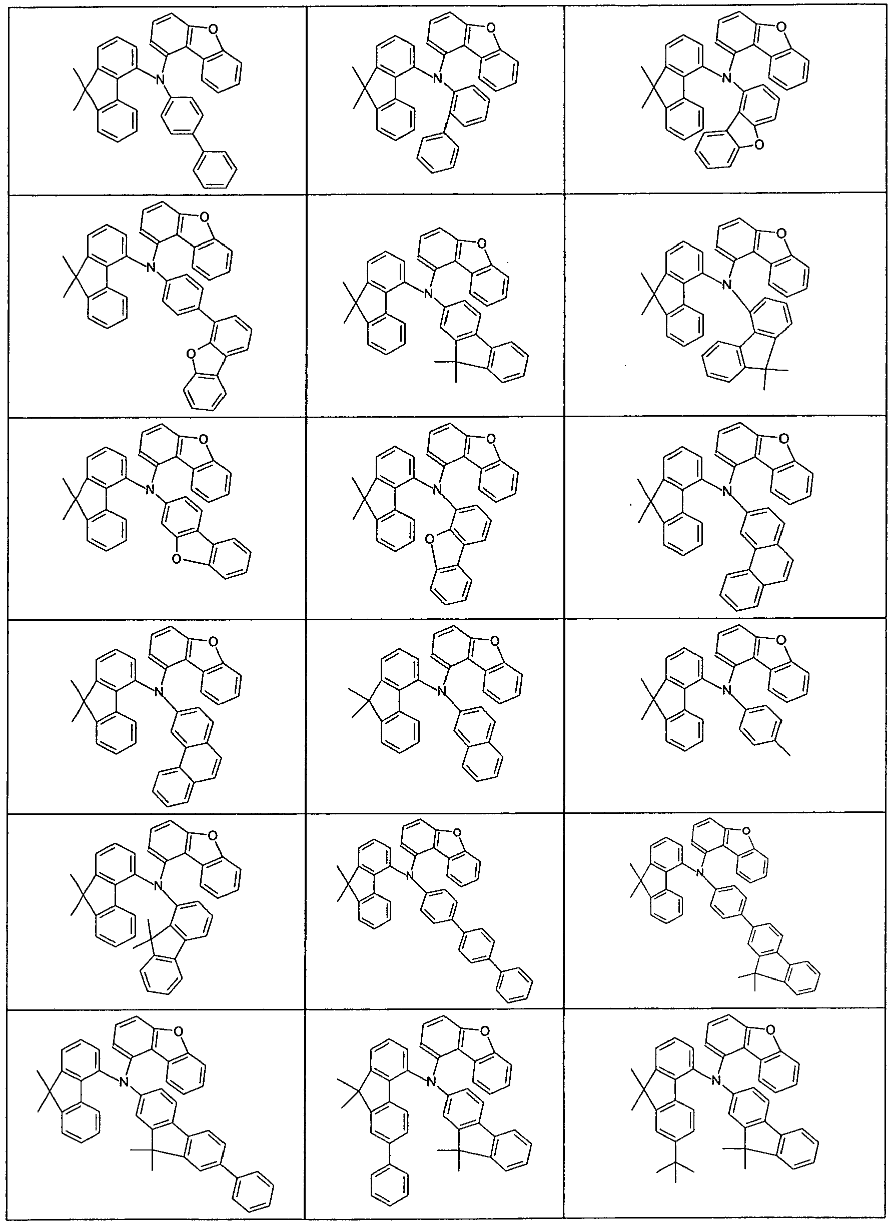

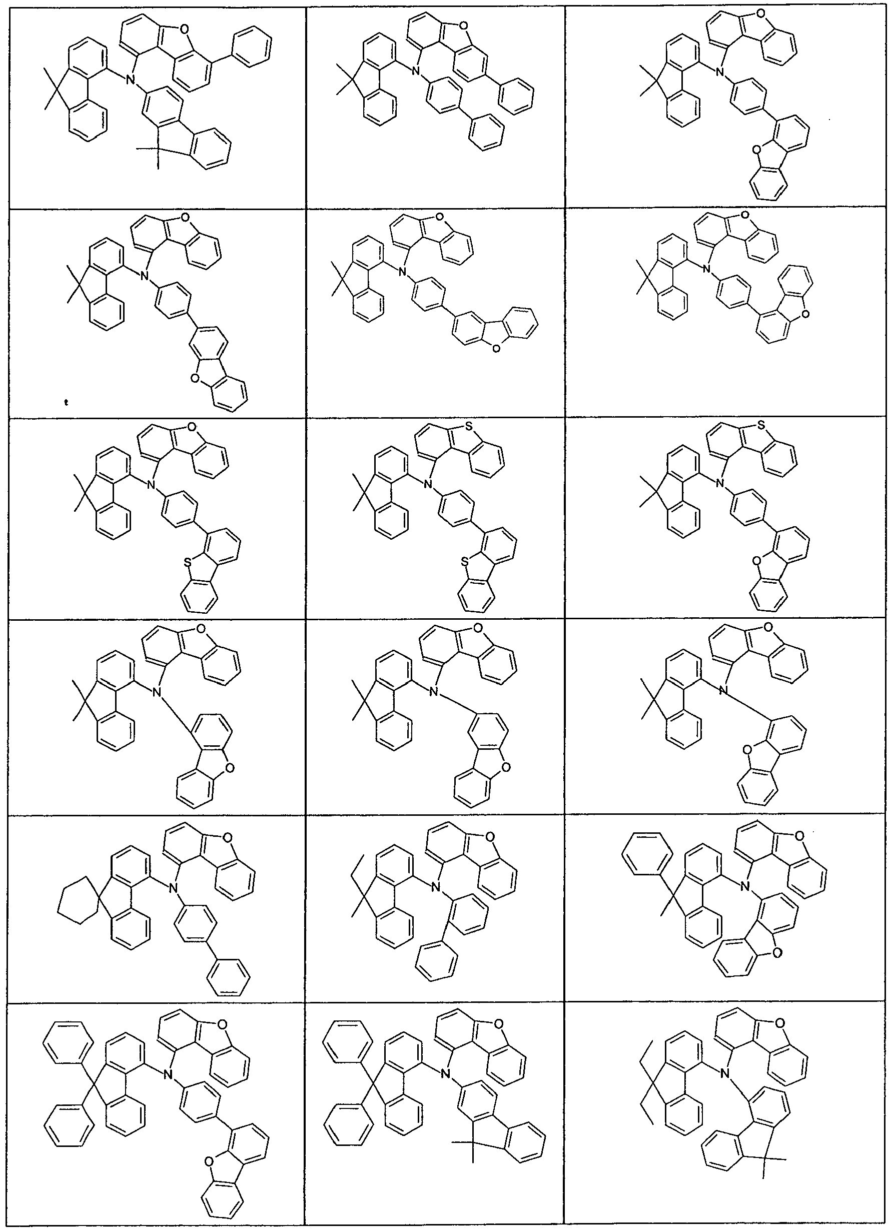

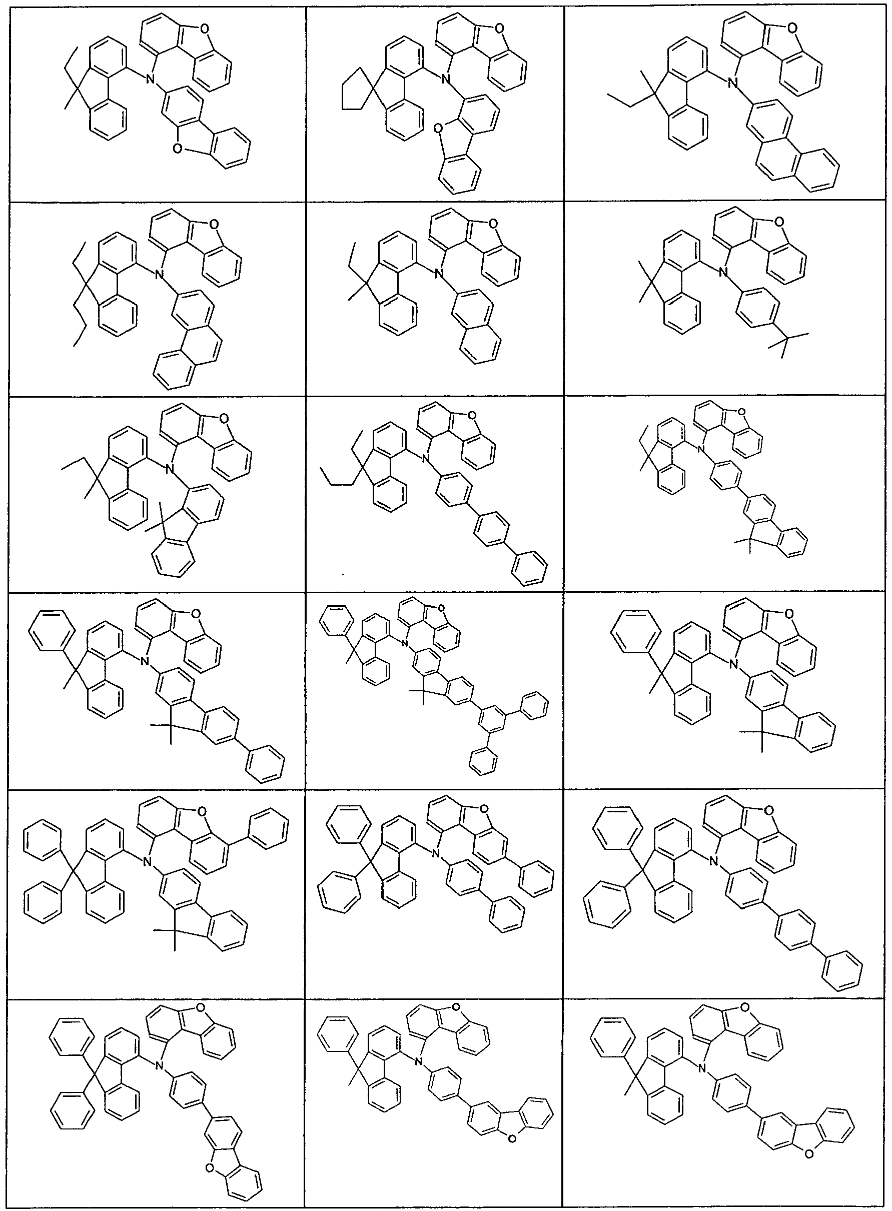

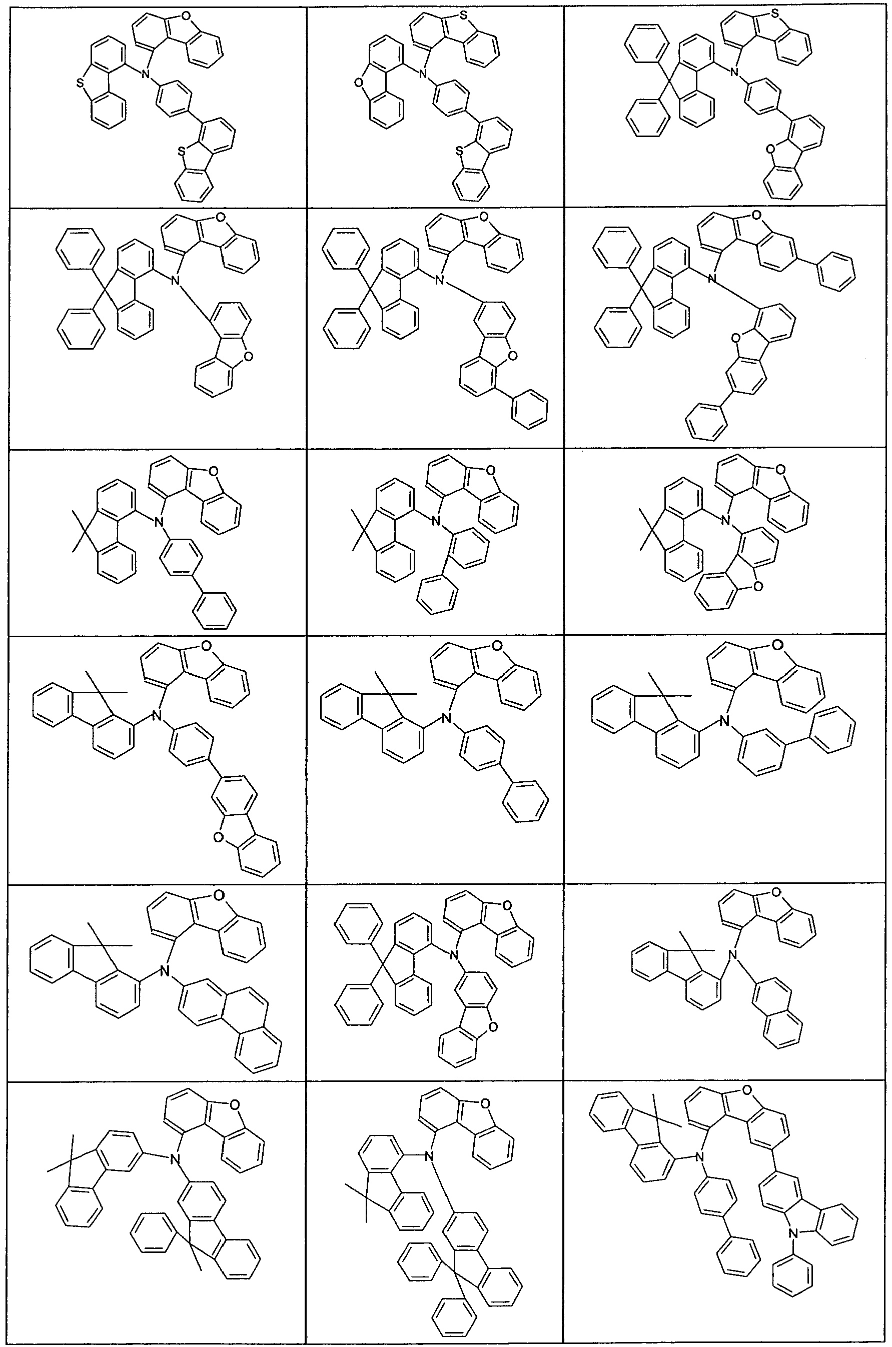

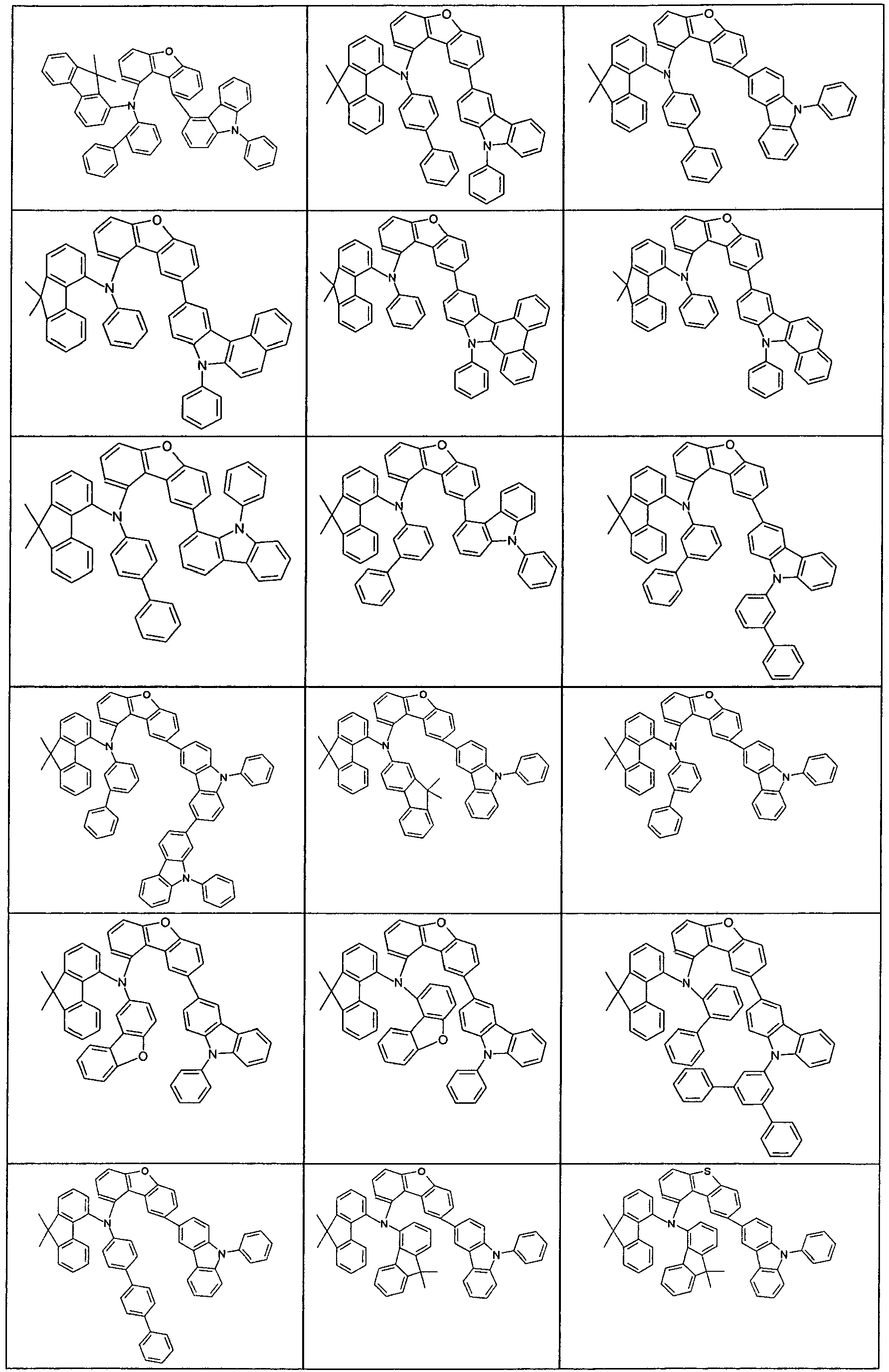

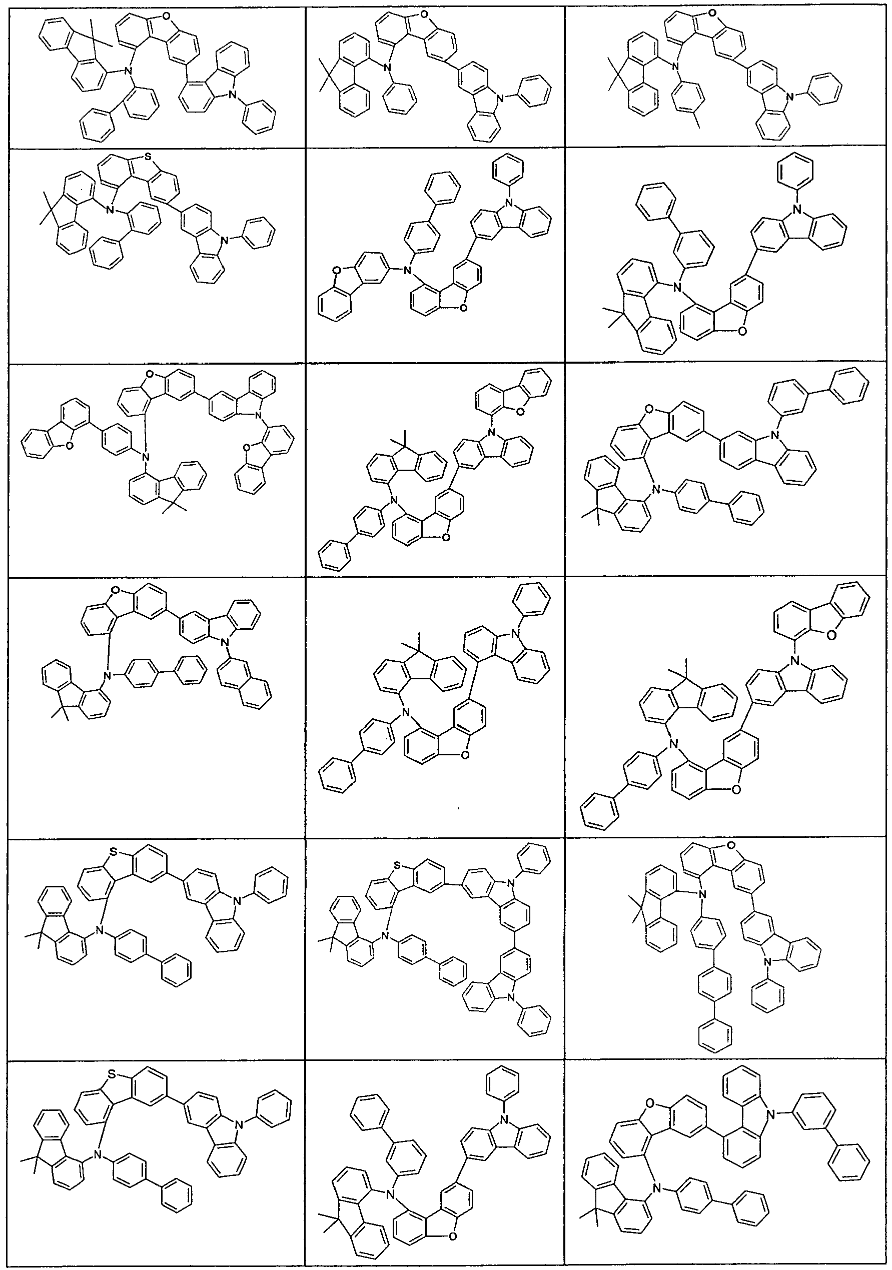

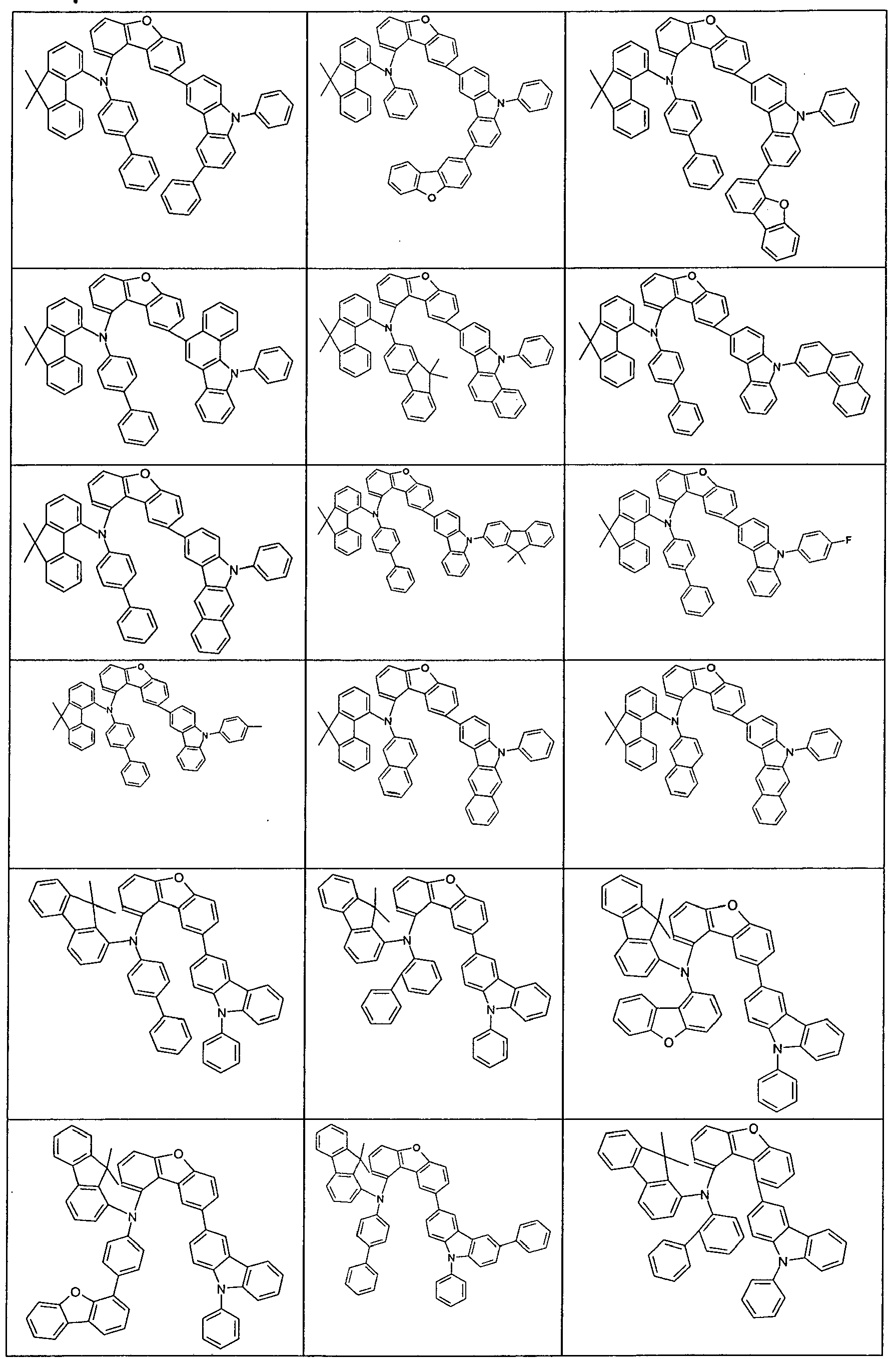

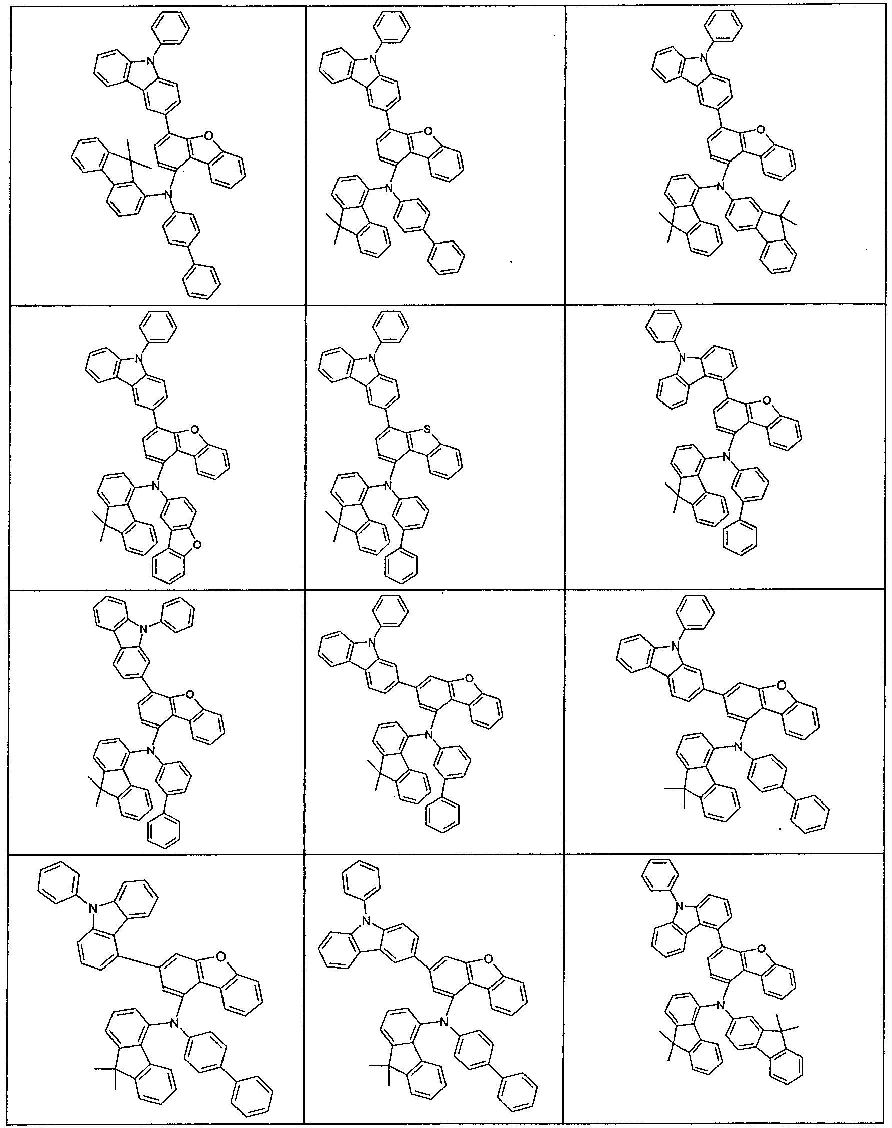

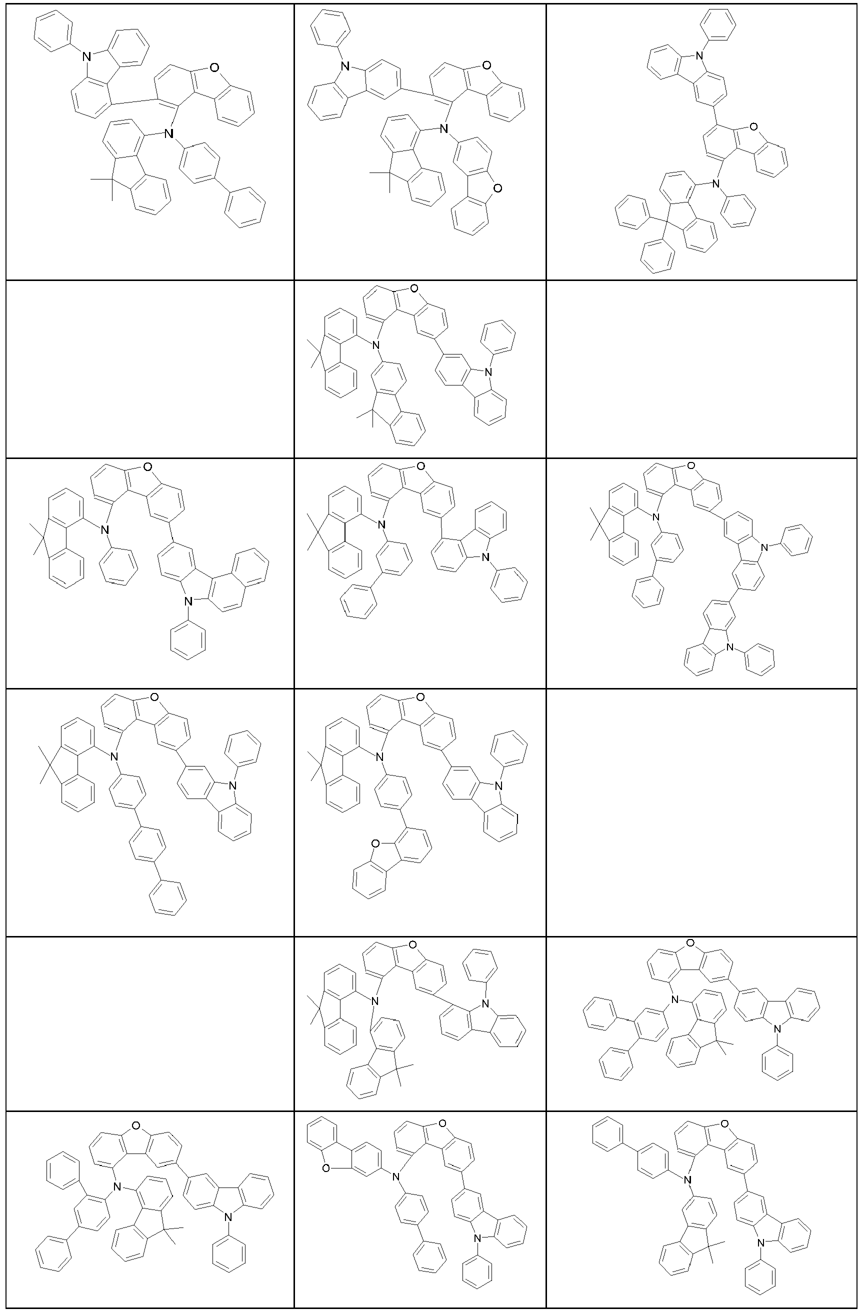

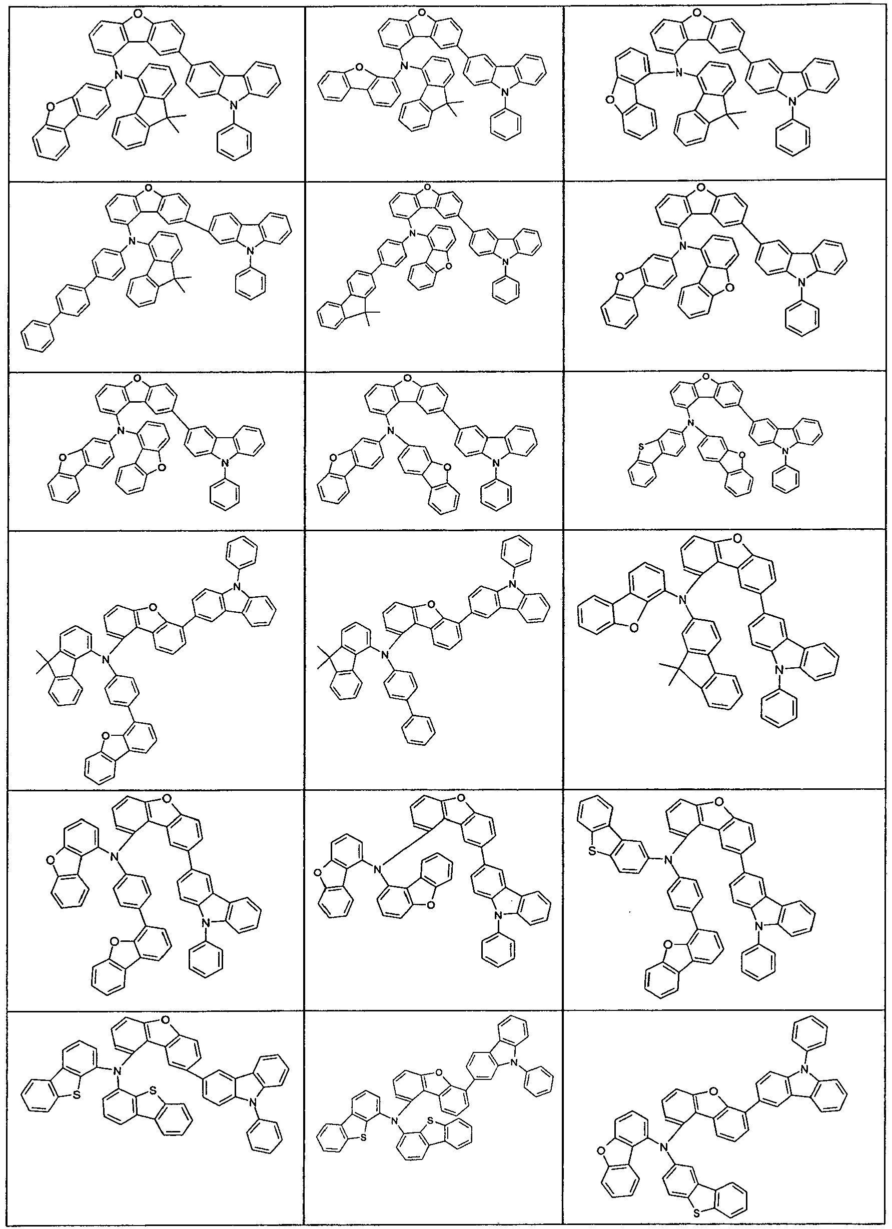

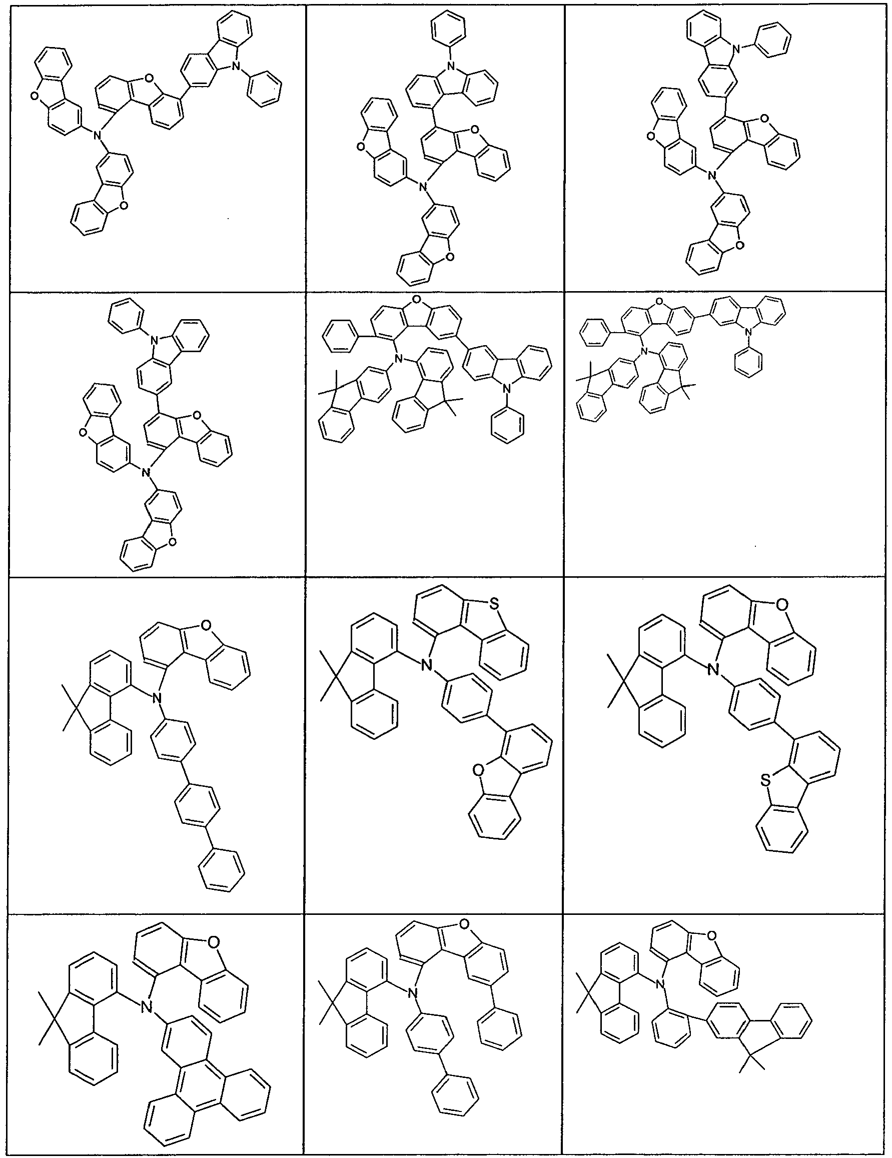

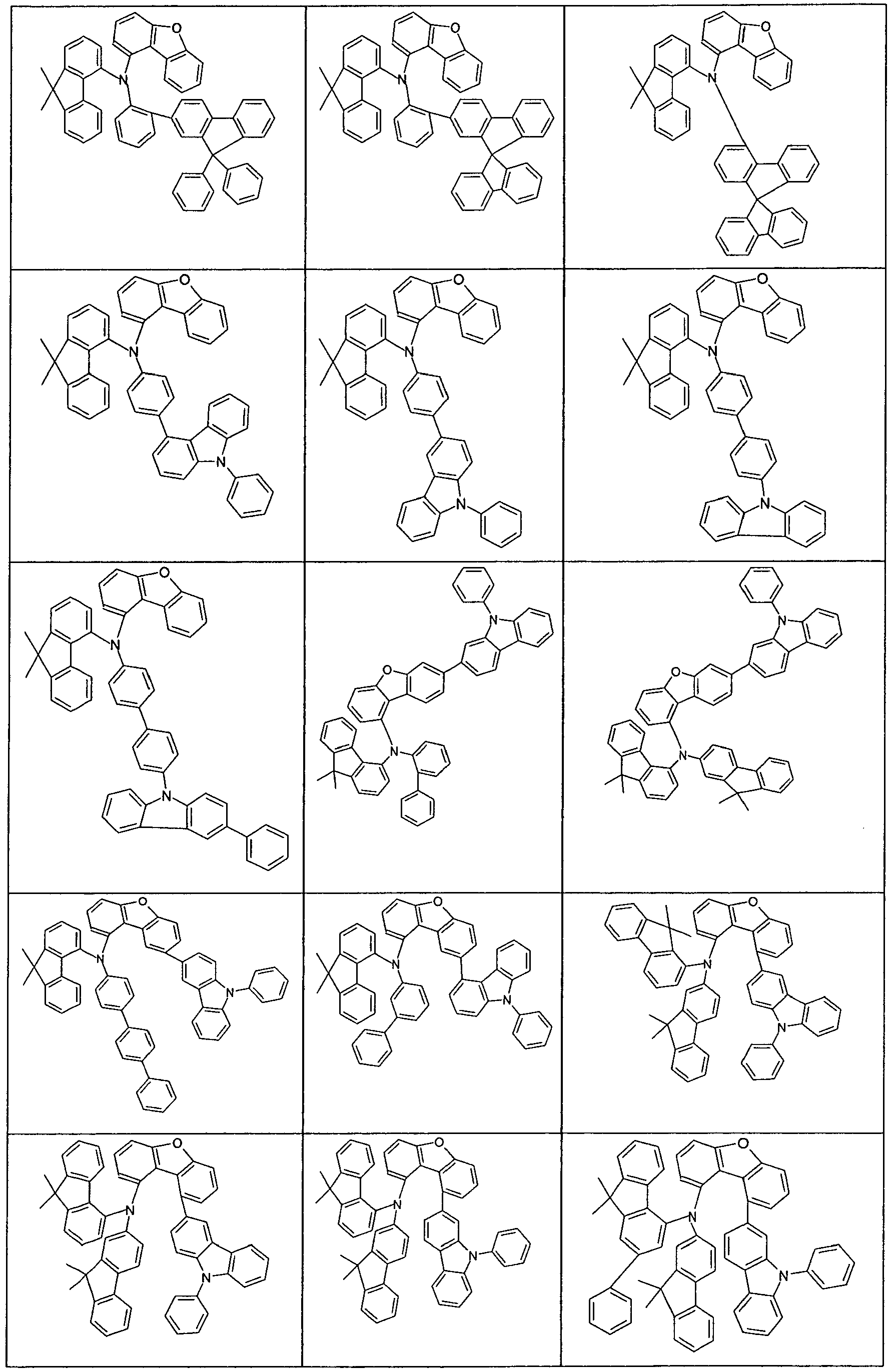

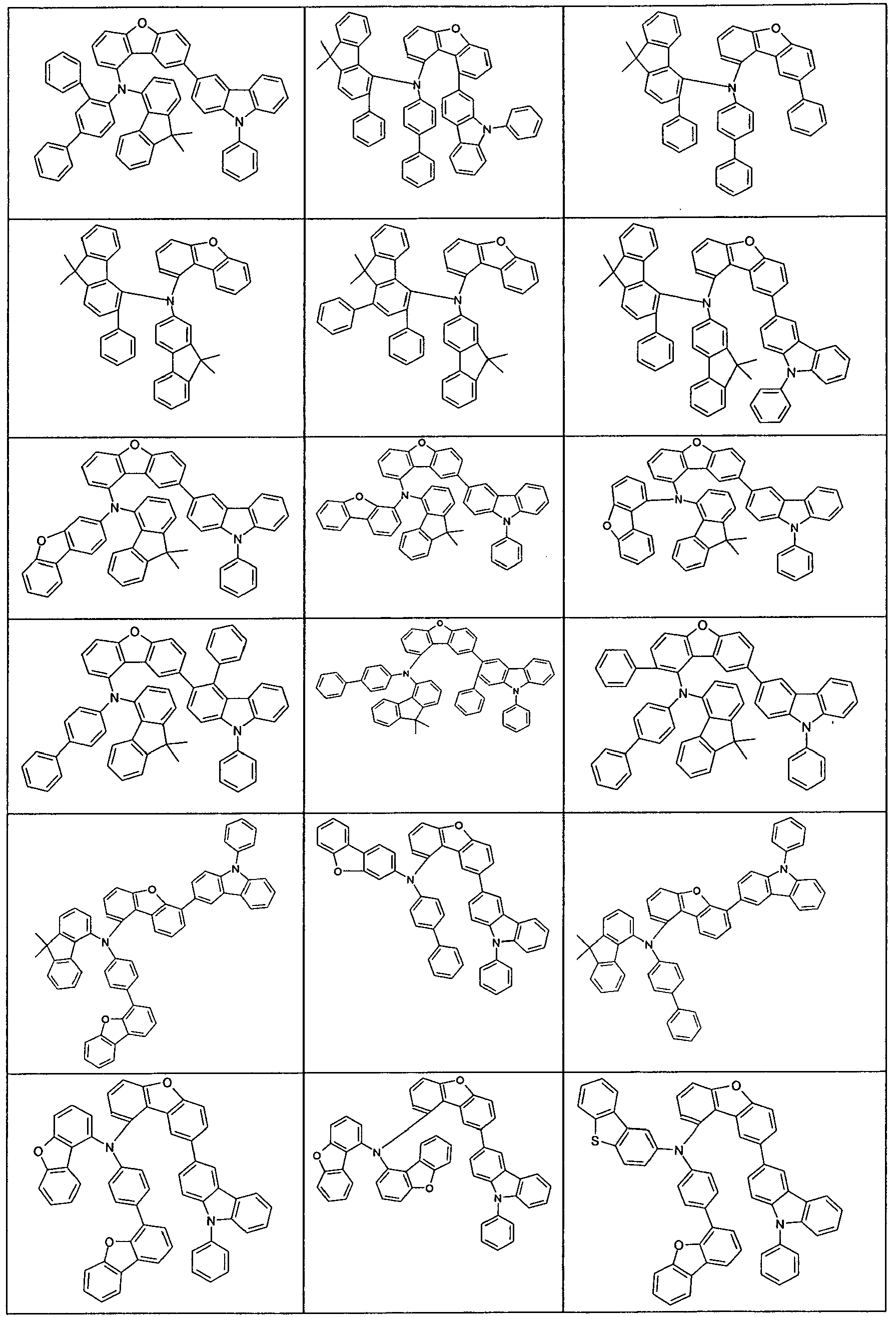

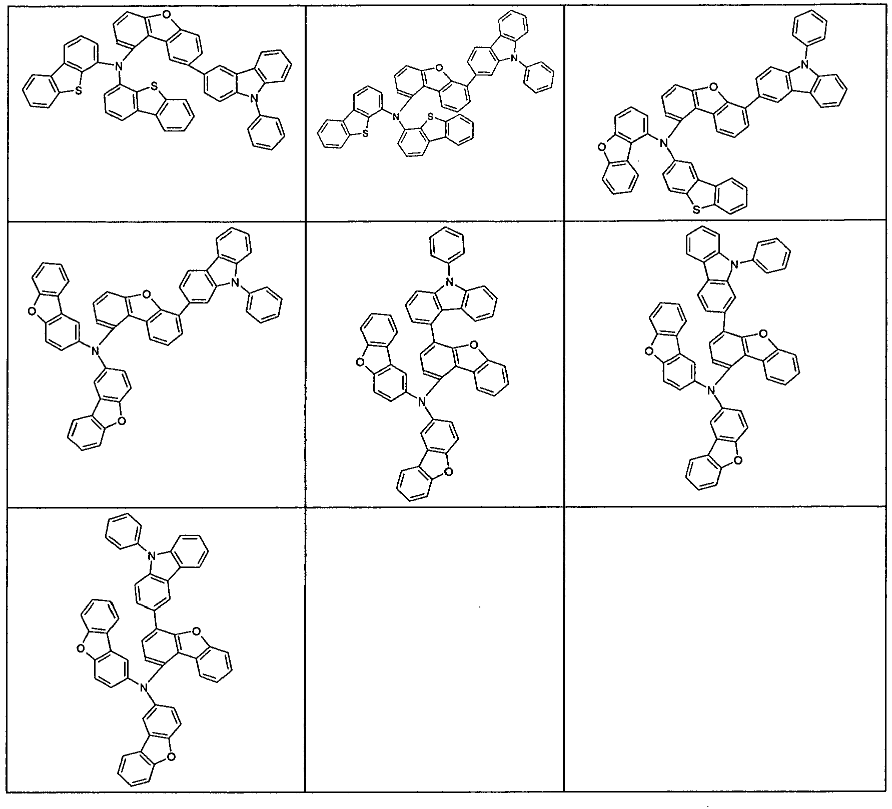

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt.

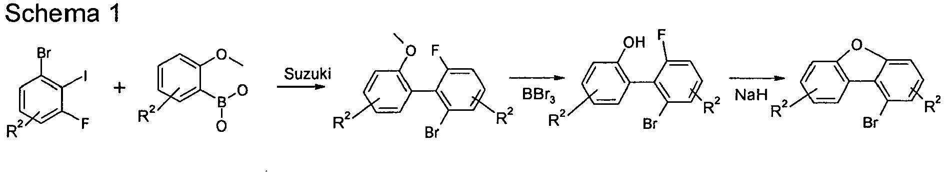

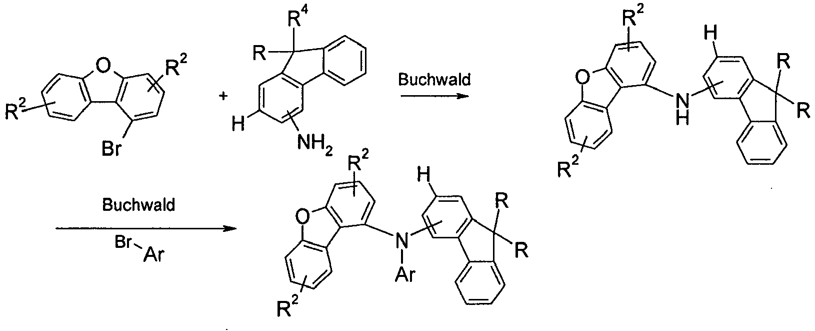

Verbindungen der Formel (2) können gemäß dem folgendem Schema (2) erhalten werden:

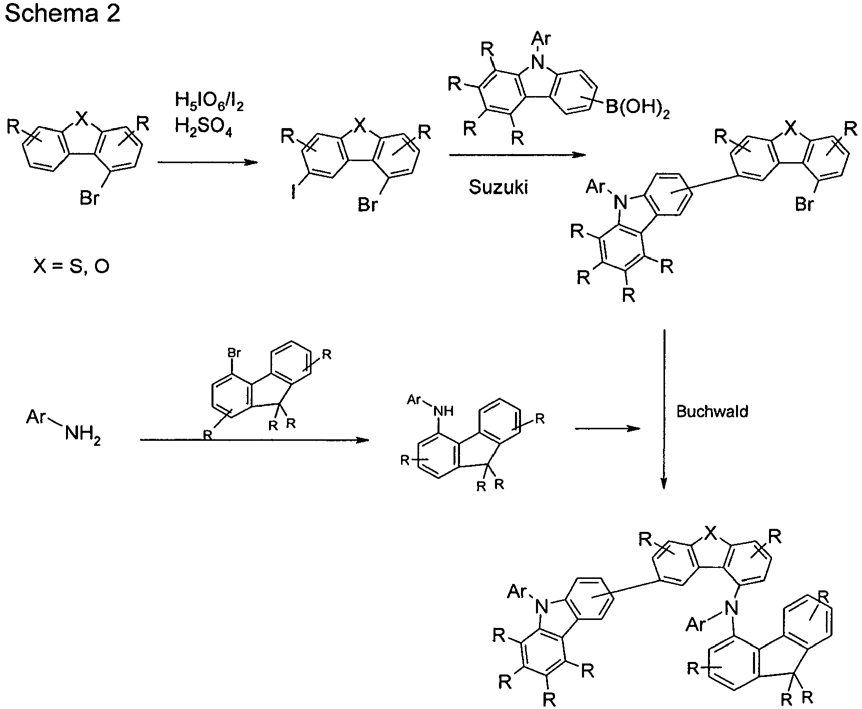

Dabei steht R entsprechend den Formeln (1) oder (2) für R, R¹, R² oder R³. Statt Fluorenderivaten können entsprechend auch Dibenzofuran- oder Dibenzothiophenderivate eingesetzt werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)-ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. In einer Ausführungsform der Erfindung handelt es sich um Systeme mit drei emittierenden Schichten, wobei die drei Schichten blaue, grüne und orange oder rote Emission zeigen. Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden. In einer weiteren Ausführungsform der Erfindung handelt es sich um eine Tandem-OLED. Eine weiß emittierende Elektrolumineszenzvorrichtung kann beispielsweise für Beleuchtungsanwendungen, aber auch in Kombination mit einem Farbfilter für Vollfarb-Displays verwendet werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter oder für Emitter, die TADF (thermally activated delayed fluorescence) zeigen, insbesondere für phosphoreszierende Emitter, und/oder in einer Elektronentransportschicht und/oder in einer elektronenblockierenden bzw. exzitonenblockierenden Schicht und/oder in einer Lochtransportschicht und/oder Lochinjektionsschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1 verstanden, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815 und WO 2016/124304 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. In einer bevorzugten Ausführungsform der Erfindung ist das weitere Matrixmaterial eine lochtransportierende Verbindung. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist.

Geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol-bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder WO 2015/192939. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransport- oder Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen, verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Die erfindungsgemäßen Verbindungen weisen eine verbesserte Oxidationsstabilität insbesondere in Lösung auf, insbesondere gegenüber üblicherweise verwendeten Diaminen. Dies ist insbesondere für Druckverfahren von Bedeutung. Die erfindungsgemäßen Verbindungen zeichnen sich außerdem durch eine hohe Temperaturstabilität aus, so dass sie im Hochvakuum unzersetzt verdampft werden können. Die Temperaturstabilität erhöht auch die operative Lebensdauer der Verbindungen.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Synthesebeispiele

### a) 4-Brom-9-methyl-9-phenyl-9H-fluoren

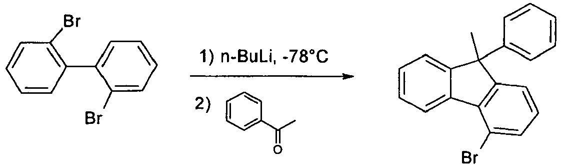

30 g (94 mmol) 2,2'-Dibrom-biphenyl werden in einem ausgeheizten Kolben in 200 mL getrocknetem THF gelöst. Die Reaktionsmischung wird auf -78 °C gekühlt. Bei dieser Temperatur werden 37.7 mL einer 2.5 M-Lösung n-Butyllithium in Hexan (94 mmol) langsam zugetropft (Dauer: ca 1 h). Der Ansatz wird 1 h bei -70°C nachgerührt. Anschließend werden 11.1 mL Acetophenon (94 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 300 ml Essigsäure versetzt, und anschließend werden 50 ml rauchende HCl zugegeben. Der Ansatz wird 6 h auf 75 °C erhitzt. Dabei fällt ein weißer Feststoff aus. Der Ansatz wird auf Raumtemperatur abgekühlt, der ausgefallene Feststoff wird abgesaugt und mit Methanol nachgewaschen. Der Rückstand wird im Vakuum bei 40 °C getrocknet. Ausbeute beträgt 25.3 g (75 mmol) (80% der Theorie).

### b) 4-Brom-9,9-diphenyl-9H-fluoren

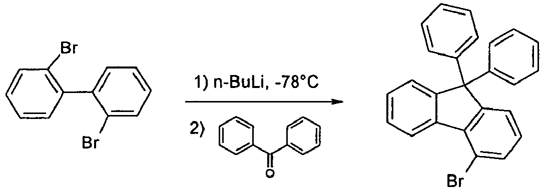

37 g (152 mmol) 2,2'-Dibrom-biphenyl werden in einem ausgeheizten Kolben in 300 mL getrocknetem THF gelöst. Die Reaktionsmischung wird auf -78 °C gekühlt. Bei dieser Temperatur werden 75 mL einer 15%-igen Lösung n-Butyllithium in Hexan (119 mmol) langsam zugetropft (Dauer: ca 1 h). Der Ansatz wird 1 h bei -70 °C nachgerührt. Anschließend werden 21.8 g Benzophenon (119 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 510 ml Essigsäure versetzt, und anschließend werden 100 ml rauchende HCl zugegeben. Der Ansatz wird 4 h auf 75 °C erhitzt. Dabei fällt ein weißer Feststoff aus. Der Ansatz wird auf Raumtemperatur abgekühlt, der ausgefallene Feststoff wird abgesaugt und mit Methanol nachgewaschen. Der Rückstand wird im Vakuum bei 40 °C getrocknet. Ausbeute beträgt 33.2 g (83 mmol) (70% der Theorie).

Analog dazu werden die folgenden bromierten Verbindungen hergestellt:

| | **Edukt 1** | **Edukt 2** | **Produkt** | **Ausbeute** |
|---|---|---|---|---|
| b1 | 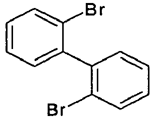 | 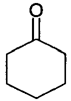 | 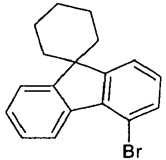 | 78% |
| b2 | 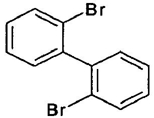 | 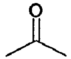 | 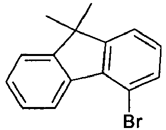 | 70% |
| b3 | 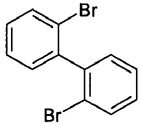 | 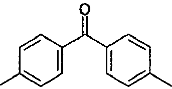 | 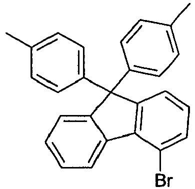 | 82% |

### c) 6-Brom-2-fluor-2'-methoxy-biphenyl

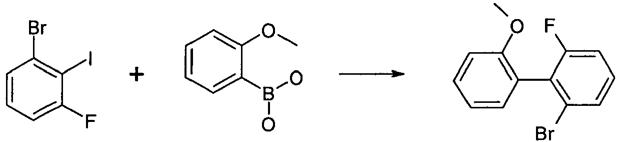

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mLTHF und 600 ml Wasser gelöst und entgast. Es wird mit 9.3 g (13,3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog dazu werden die folgenden Verbindungen hergestellt:

| | **Edukt** 1 | **Edukt 2** | **Produkt** | **Ausbeute** |
|---|---|---|---|---|
| c1 | 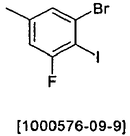 | 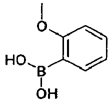 | 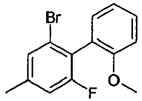 | 77% |
| c2 | 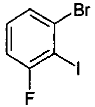 | 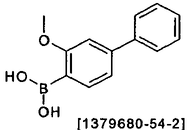 | 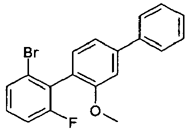 | 74% |
| c3 | 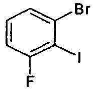 | 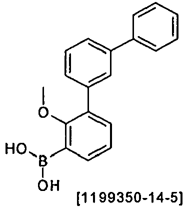 | 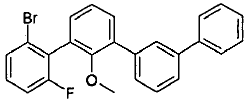 | 76% |
| c3 | 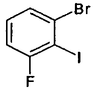 | 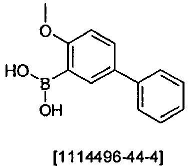 | 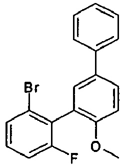 | 71% |

### d) 6'-Brom-2'-fluor-biphenyl-2-ol

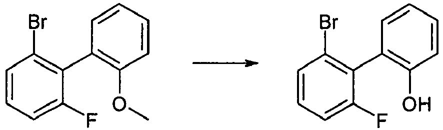

112 g (418 mmol) 6-Brom-2-fluor-2'-methoxy-biphenyl werden in 2 L Dichloromethan gelöst und auf 5 °C gekühlt. Zu dieser Lösung wird innerhalb von 90 min. 41.0 ml (431 mmol) Bortribromid zugetropft und über Nacht weiter gerührt. Das Gemisch wird im Anschluss langsam mit Wasser versetzt, die organische Phase dreimal mit Wasser gewaschen, über Na₂SO₄ getrocknet, einrotiert und chromatographisch gereinigt. Ausbeute: 104 g (397 mmol), 98 % der Theorie.

Analog dazu werden die folgenden Verbindung hergestellt:

| | **Edukt 1** | **Produkt** | **Ausbeute** |
|---|---|---|---|
| d1 | 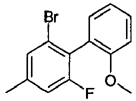 | 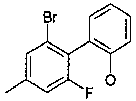 | 92% |
| d2 | 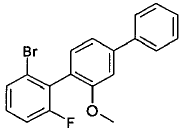 | 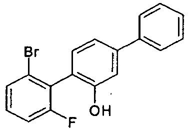 | 90% |
| d3 | 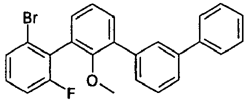 | 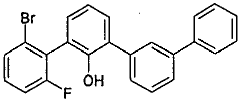 | 93% |
| d4 | 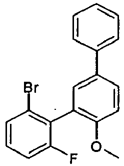 | 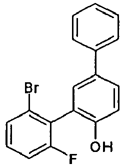 | 94% |

### e) 1-Brom-dibenzofuran

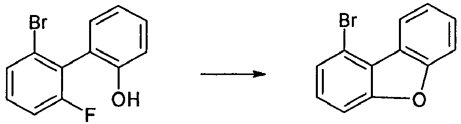

111 g (416 mmol) 6'-Brom-2'-fluor-biphenyl-2-ol werden in 2 L DMF (max. 0.003 % H₂O) SeccoSolv® gelöst und auf 5 °C gekühlt. Zu dieser Lösung wird portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und dann chromatographisch gereinigt. Ausbeute: 90 g (367 mmol), 88,5 % der Theorie.

Analog dazu werden die folgenden Verbindungen hergestellt:

| | **Edukt 1** | **Produkt** | **Ausbeute** |
|---|---|---|---|
| e1 | 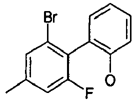 | 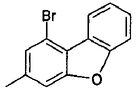 | 81% |
| e2 | 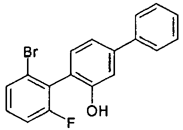 | 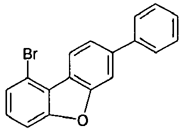 | 78% |
| e3 | 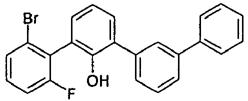 | 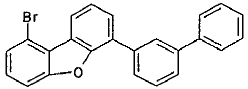 | 73% |
| e4 | 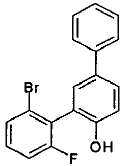 | 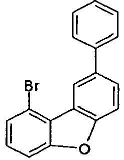 | 79% |

### f) Biphenyl-4-yl-dibenzofuran-1-yl-amin

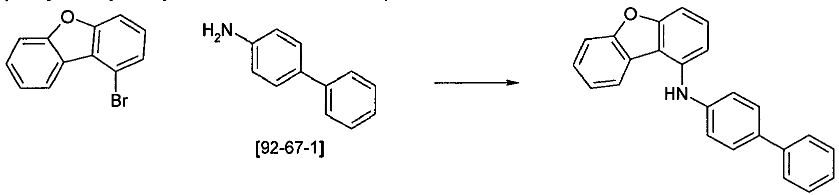

30.0 g (177 mmol, 1.0 eq) 4-Aminobiphenyl werden zusammen mit 43.7 g (177 mmol, 1.0 eq) 1-Bromdibenzofuran und 2.4 g (212 mmol, 1.20 eq) Natrium-*tert*-pentoxid [14593-46-5] in 600 ml absolutem Toluol vorgelegt und für 30 Minuten entgast. Anschließend werden 398 mg (1.77 mmol, 0.01 eq) Palladium(II)-acetat [3375-31-3] und 1.46 g (3.56 mmol, 0.02 eq) 2-Dicyclohexylphosphino-2',6'-dimethoxybiphenyl SPHOS [657408-07-6] zugegeben und der Ansatz über Nacht unter Rückfluss erhitzt. Nach beendeter Reaktion wird der Ansatz auf Raumtemperatur abgekühlt und mit 500 ml Wasser extrahiert. Anschließend wird die wässrige Phase dreifach mit Toluol gewaschen, die vereinten organischen Phasen über Natriumsulfat getrocknet und das Lösungsmittel am Rotationsverdampfer entfernt. Der braune Rückstand wird in ca. 200 ml Toluol aufgenommen und über Silicagel filtriert. Zur weiteren Aufreinigung wird eine Umkristallisation aus Toluol/Heptan durchgeführt. Ausbeute: 44 g (133 mmol), 76 % der Theorie.

Analog dazu werden die folgenden Verbindungen hergestellt:

| | **Edukt 1** | **Edukt 2** | **Produkt 3** | **Ausbeute [%]** |
|---|---|---|---|---|
| f1 | 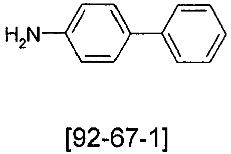 | 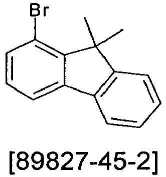 | 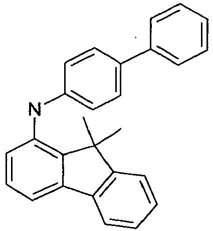 | 45 |
| f2 | 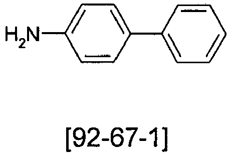 | 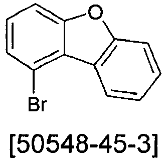 | 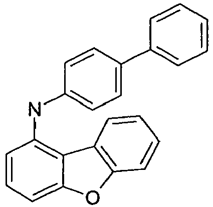 | 61 |
| f3 | 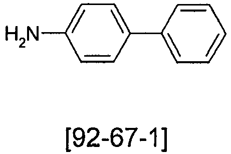 | 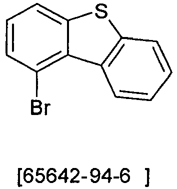 | 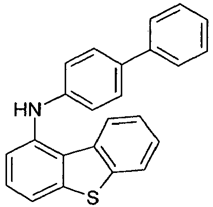 | 68 |
| f4 | 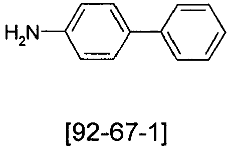 | 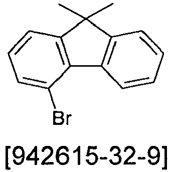 | 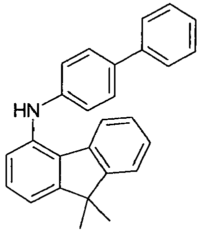 | 34 |
| f5 | 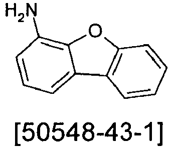 | 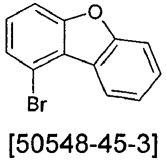 | 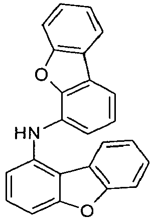 | 73 |
| f6 | 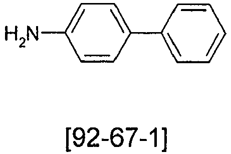 | 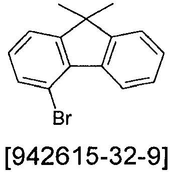 | 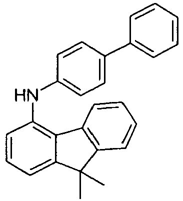 | 51 |
| f7 | 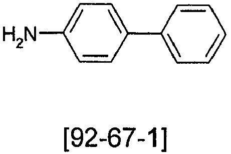 | 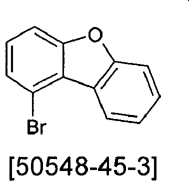 | 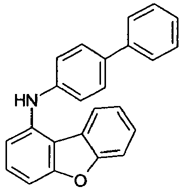 | 65 |
| f8 | 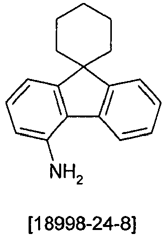 | 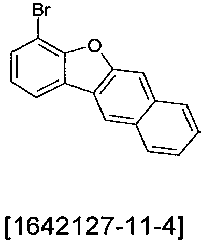 | 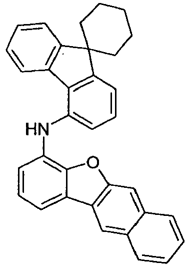 | 81 |
| f9 | 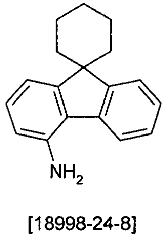 | 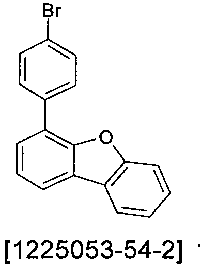 | 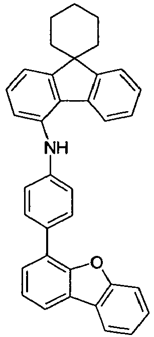 | 62 |
| f10 | 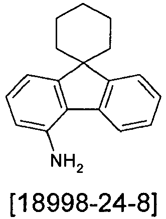 | 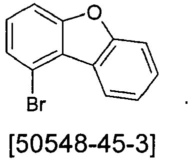 | 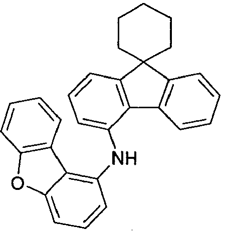 | 43 |
| f11 | 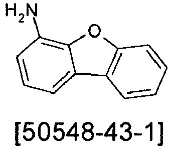 | 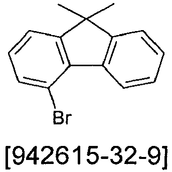 | 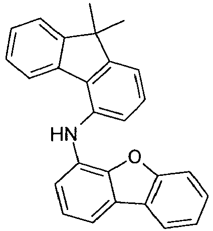 | 34 |
| f12 | 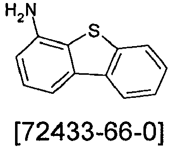 | 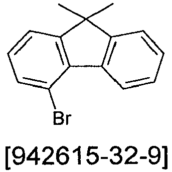 | 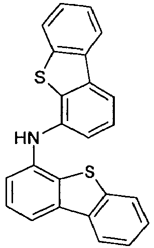 | 55 |
| f13 | 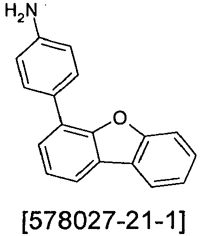 | 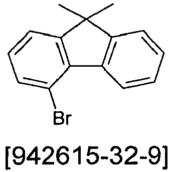 | 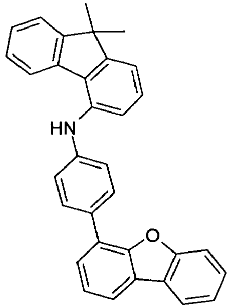 | 62 |
| f14 | 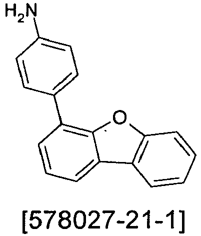 | 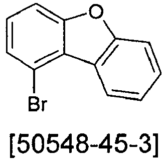 | 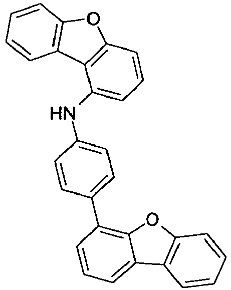 | 70 |
| f15 | 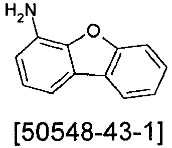 | 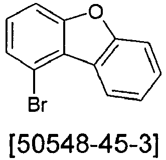 | 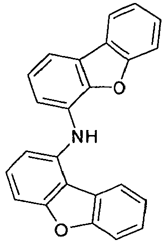 | 77 |
| f16 | 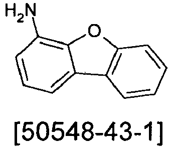 | 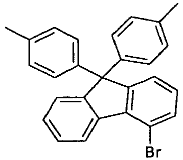 | 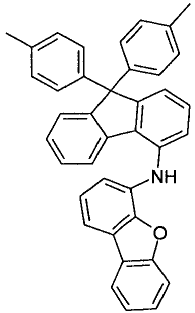 | 75 |

### g) Dibenzofuran-1-yl-(4-dibenzofuran-4-yl-phenyl)-(9,9-dimethyl-9H-fluoren-4-yl)-amin

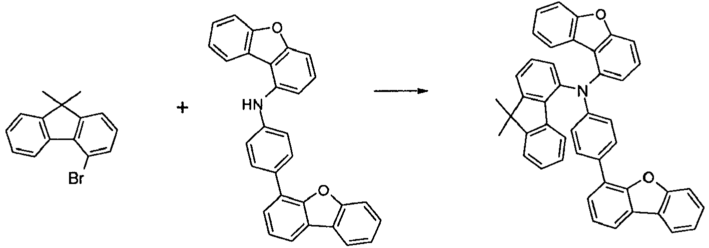

Ein Gemisch aus 13.6 g (50 mmol) 4-Bromo-9,9-dimethyl-9H-fluoren, 25.5 g (60 mmol) Dibenzofuran-1-yl-(4-dibenzofuran-4-yl-phenyl)-amin, 7.7 g (80 mmol) Natrium-tert-butylat, 1.4 g (5 mmol) Tricyclohexylamin, 561 mg (2.5 mmol) Palladium(II)acetat und 300 ml Mesitylen wird 24 h unter Rückfluss erhitzt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 340-350 °C). Ausbeute: 23 g (37 mmol), 75 % der Theorie: 99.9 % n. HPLC.

Analog werden folgende Verbindungen erhalten:

| **Bsp.** | **Edukt 1** | **Edukt 2** | **Produkt** | **Ausbeute [%]** |
|---|---|---|---|---|
| g1 | 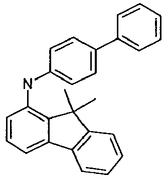 | 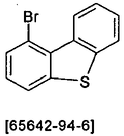 | 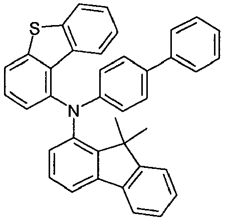 | 69 |
| g2 | 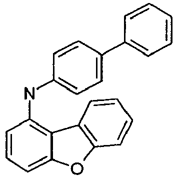 | 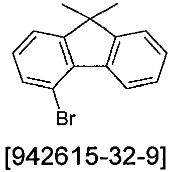 | 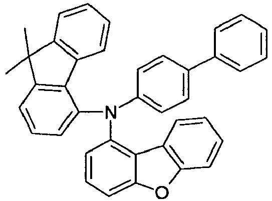 | 72 |
| g3 | 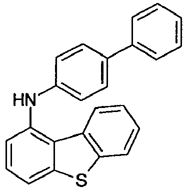 | 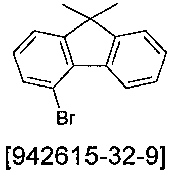 | 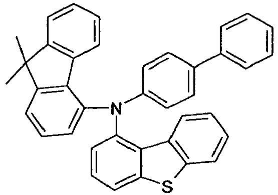 | 71 |
| g4 | 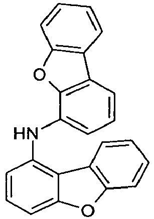 | 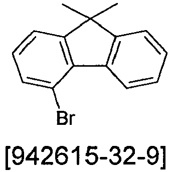 | 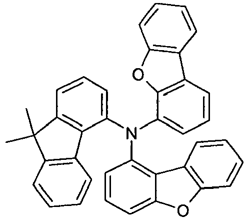 | 69 |
| g5 | 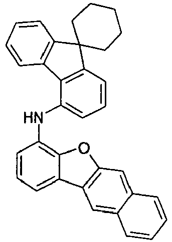 | 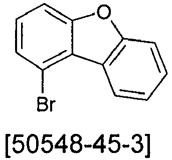 | 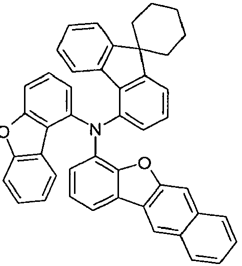 | 74 |
| g6 | 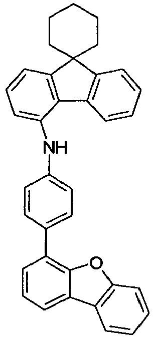 | 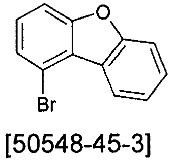 | 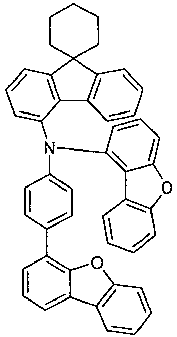 | 75 |
| g7 | 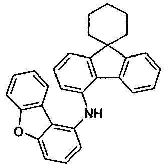 | 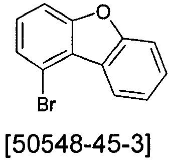 | 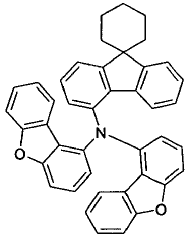 | 72 |
| g8 | 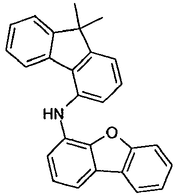 | 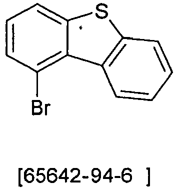 | 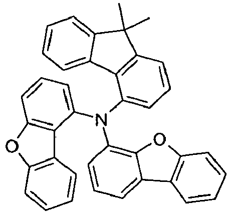 | 70 |
| g9 | 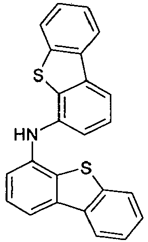 | 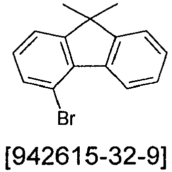 | 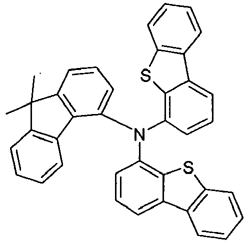 | 71 |
| g11 | 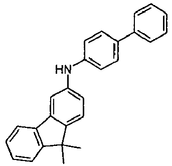 | 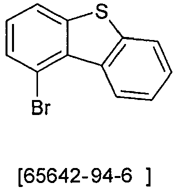 | 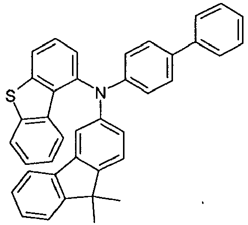 | 68 |
| g12 | 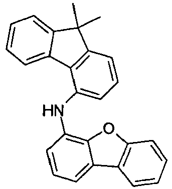 | 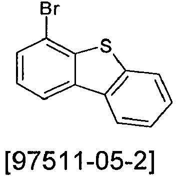 | 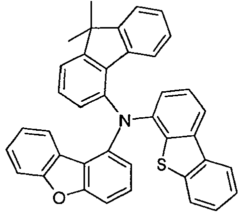 | 75 |
| g15 | 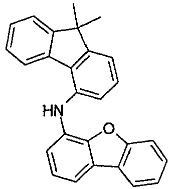 | 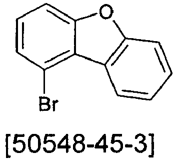 | 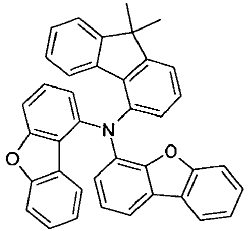 | 63 |
| g16 | 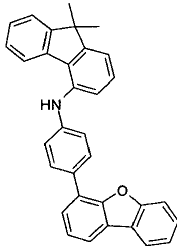 | 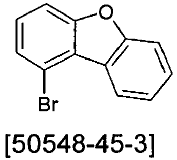 | 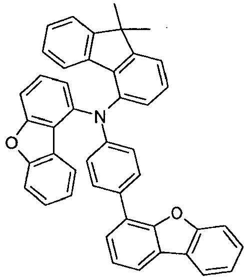 | 73 |
| g17 | 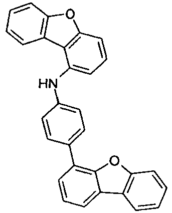 | 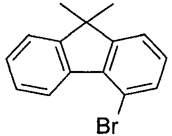 | 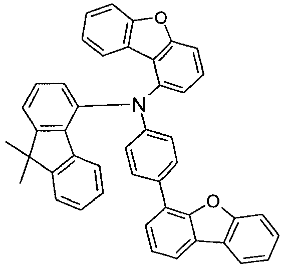 | 72 |
| g18 | 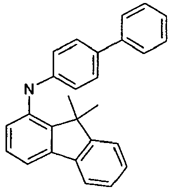 | 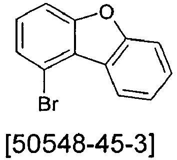 | 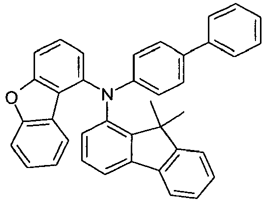 | 77 |
| g19 | 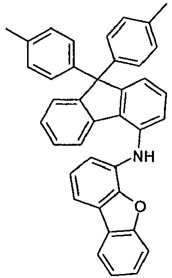 | 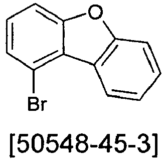 | 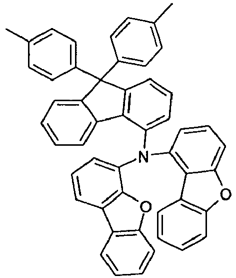 | 72 |
| g20 | 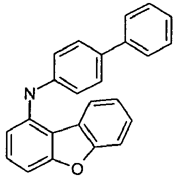 | 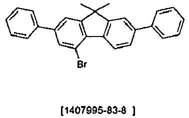 | 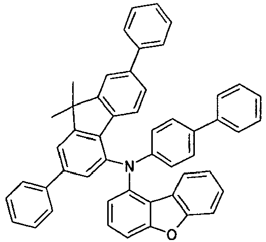 | 63 |
| g21 | 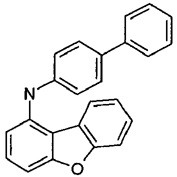 | 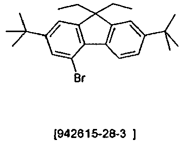 | 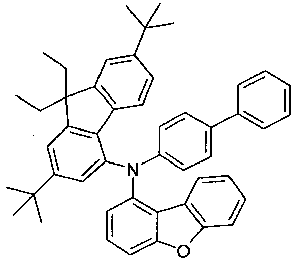 | 67 |
| g22 | 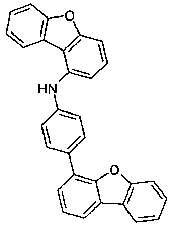 | 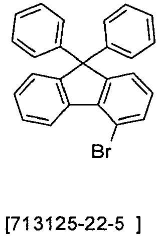 | 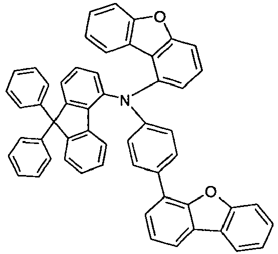 | 60 |

### h) 1-Bromo-8-iodo-dibenzofuran

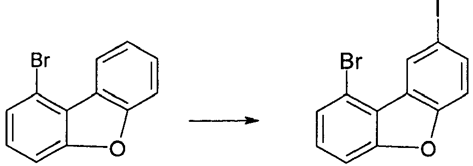

20 g (80 mmol) Dibenzofuran-1-boronsäure, 2.06 g (40.1 mmol) Iod, 3.13 g (17.8 mmol) Iodsäure, 80 ml Essigsäure und 5 ml Schwefelsäure und 5 ml Wasser und 2 ml Chloroform werden 3 h bei 65 °C gerührt. Nach Erkalten wird die Mischung mit Wasser versetzt, der ausgefallene Feststoff abgesaugt und dreimal mit Wasser gewaschen. Der Rückstand wird aus Toluol und aus Dichlormethan /Heptan umkristallisiert. Die Ausbeute beträgt 25.6 g (68 mmol), entsprechend 85 % der Theorie.

Analog dazu werden die folgenden Verbindungen hergestellt:

| | **Edukt 1** | **Produkt** | **Ausbeute** |
|---|---|---|---|
| h1 | 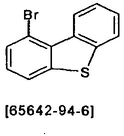 | 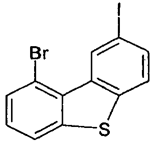 | 81% |
| h2 | 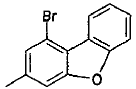 | 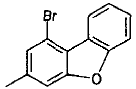 | 67% |

### i) 3-(9-Bromo-dibenzofuran-2-yl)-9-phenyl-9-H-carbazol

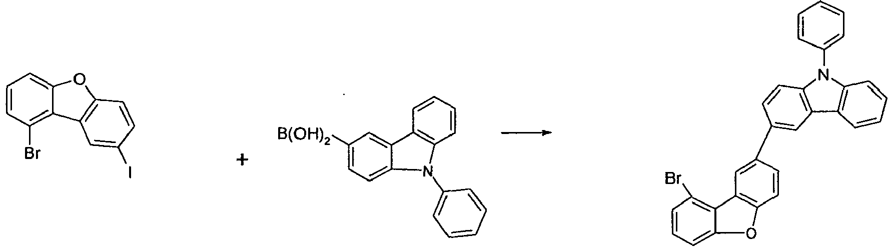

58 g (156 mmol) 1-Bromo-8-iodo-dibenzofuran, 50 g (172 mmol) N-Phenyl-carbazol-3-boronsäure und 36 g (340 mmol) Natriumcarbonat werden in 1000 mL Ethylenglycoldimethylether und 280 mL Wasser suspendiert. Zu dieser Suspension werden 1.8 g (1.5 mmol) Tetrakis(triphenylphosphin)-palladium(0)zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 48 g (89 mmol), entsprechend 64 % der Theorie.

Analog dazu werden die folgendenVerbindungen hergestellt:

| | **Edukt 1** | **Edukt 2** | **Produkt** | **Ausbeute** |
|---|---|---|---|---|
| i1 | 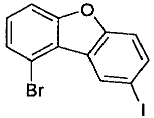 | 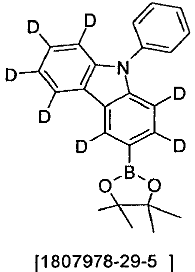 | 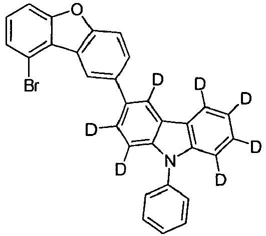 | 67% |
| i2 | 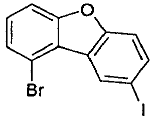 | 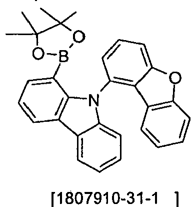 | 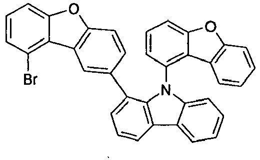 | 65% |
| i3 | 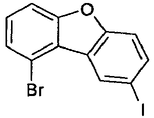 | 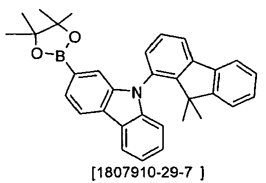 | 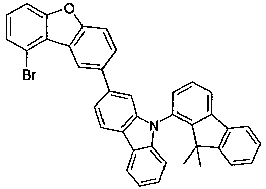 | 60% |
| i4 | 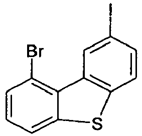 | 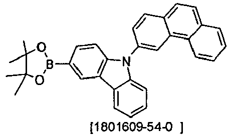 | 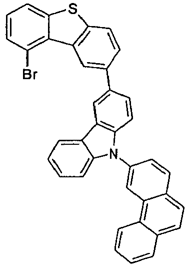 | 63% |
| i5 | 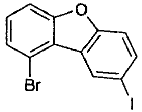 | 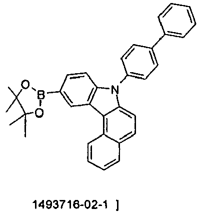 | 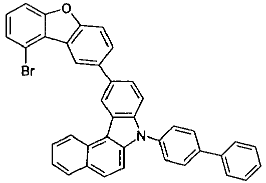 | 61% |
| i6 | 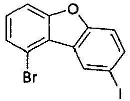 | 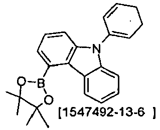 | 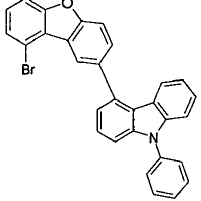 | 60% |
| i7 | 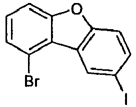 | 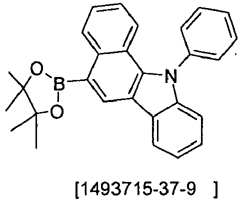 | 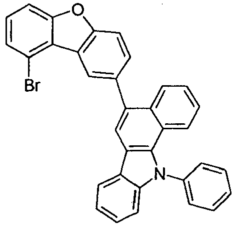 | 56% |
| i8 | 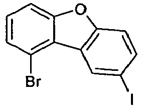 | 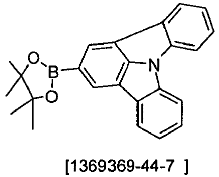 | 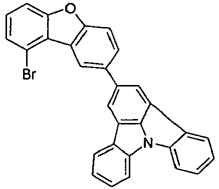 | 54% |
| i9 | 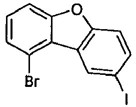 | 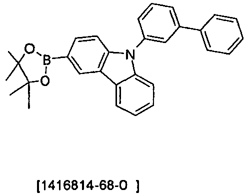 | 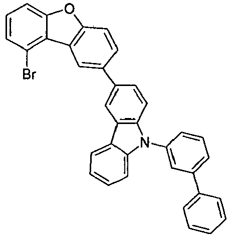 | 68% |
| i10 | 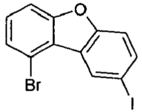 | 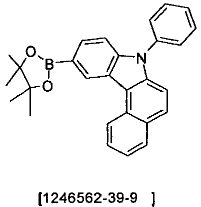 | 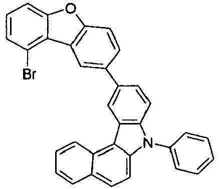 | 67% |
| i11 | 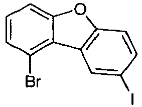 | 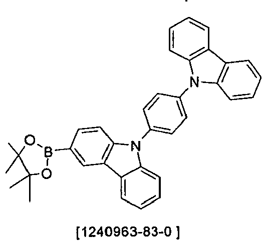 | 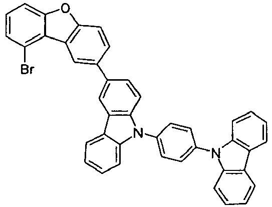 | 66% |
| i12 | 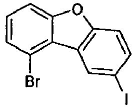 | 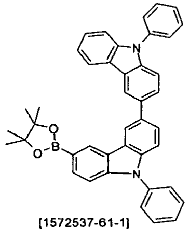 | 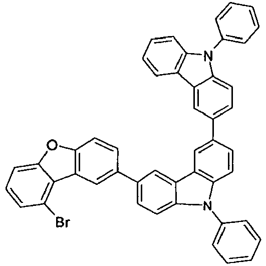 | 60% |
| i13 | 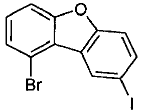 | 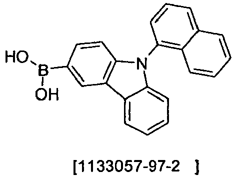 | 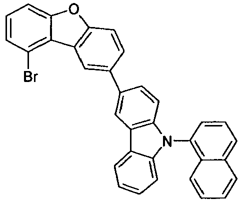 | 63% |
| i14 | 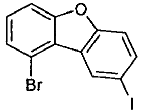 | 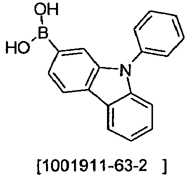 | 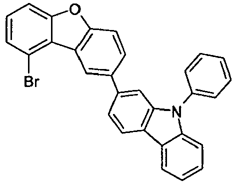 | 62% |
| i15 | 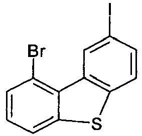 | 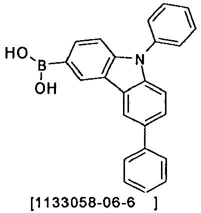 | 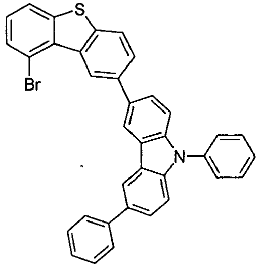 | 59% |
| i16 | 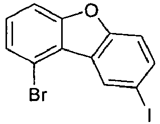 | 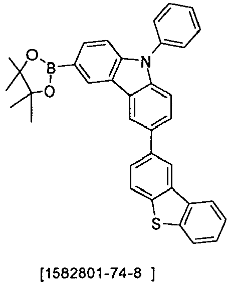 | 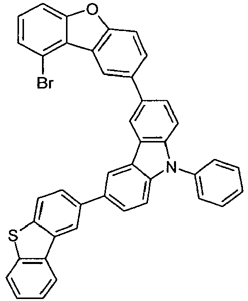 | 65% |
| i17 | 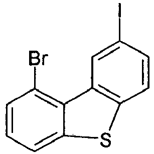 | 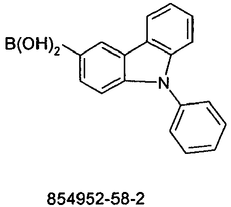 | 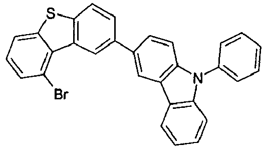 | 57% |

### j) Biphenyl-4-yl-(9,9-dimethyl-9H-fluoren-4-yl)-[8-(9-phenyl-9H-carbazol-3-yl)-dibenzofuran-1-yl]-amin

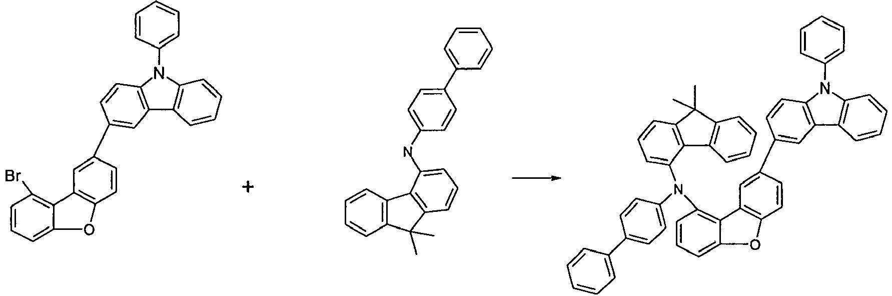

Ein Gemisch aus 9.3 g (26 mmol) Biphenyl-4-yl-(9,9-dimethyl-9H-fluoren-4-yl)-amin, 12 g (26 mmol)3-(9-Brom-dibenzofuran-2-yl)-9-phenyl-9-H-Carbazol, 7.7 g (80 mmol) Natrium-tert-butylat, 2.6 ml (78 mmol) Tri-tert-butylphosphin (1M, Toluol), 224 mg (2.6 mmol) Palladium(II)acetat und 300 ml Mesitylen wird 24 h unter Rückfluss erhitzt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 340-350 °C). Ausbeute: 13 g (17 mmol), 68 % der Theorie: 99.9 % n. HPLC.

Analog dazu werden die folgenden Verbindungen hergestellt:

| | **Edukt 1** | **Edukt 2** | **Produkt** | **Ausbeute** |
|---|---|---|---|---|
| j1 | 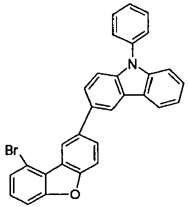 | 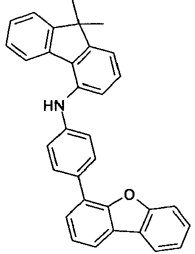 | 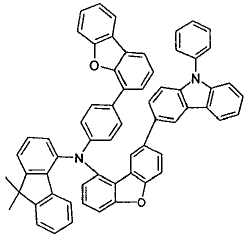 | 61% |
| j2 | 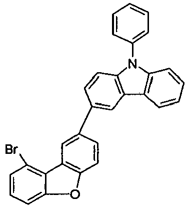 | 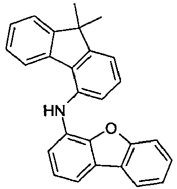 | 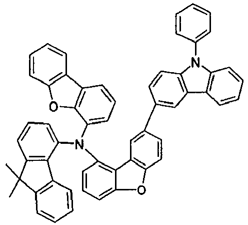 | 65% |
| j3 | 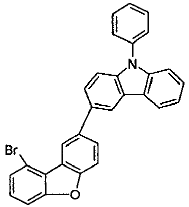 | 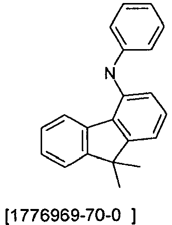 | 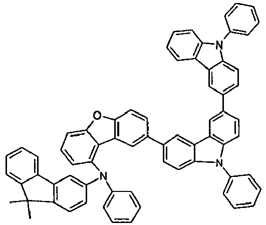 | 57% |
| j4 | 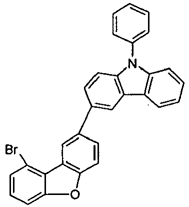 | 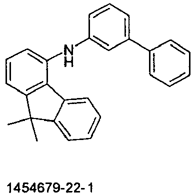 | 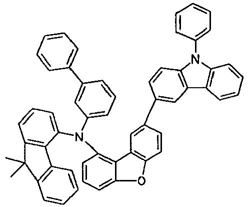 | 72% |
| j5 | 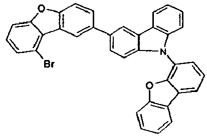 | 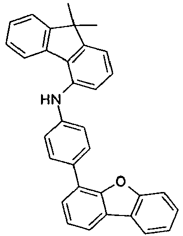 | 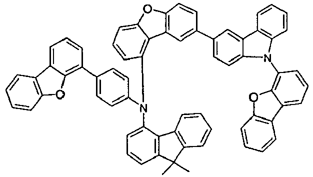 | 63% |
| j6 | 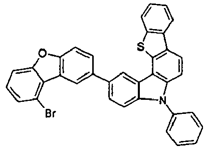 | 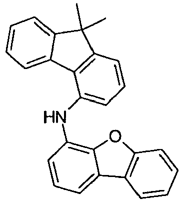 | 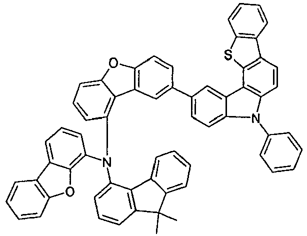 | 62% |
| j7 | 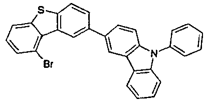 | 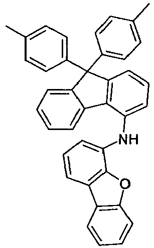 | 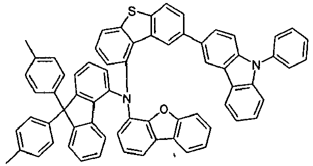 | 54% |
| j8 | 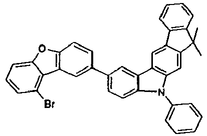 | 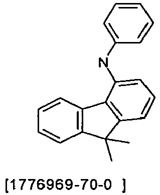 | 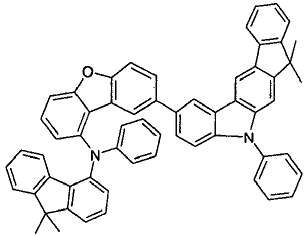 | 51% |
| j9 | 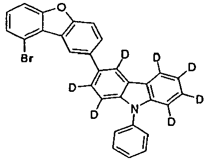 | 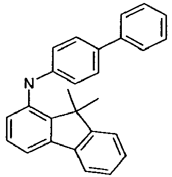 | 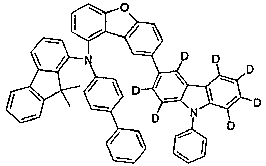 | 53% |
| j10 | 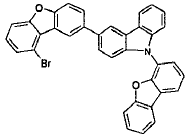 | 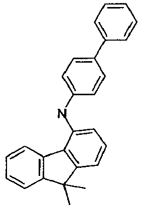 | 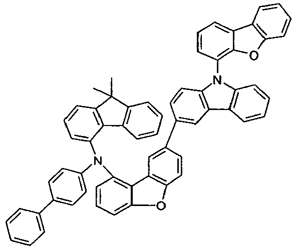 | 56% |
| j11 | 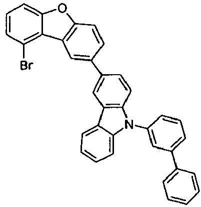 | 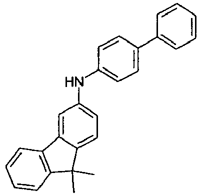 | 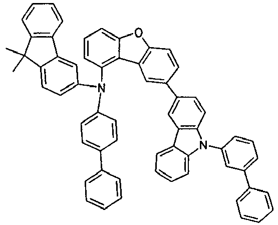 | 66% |
| j12 | 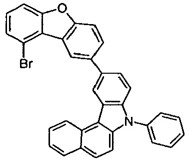 | 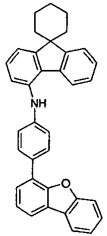 | 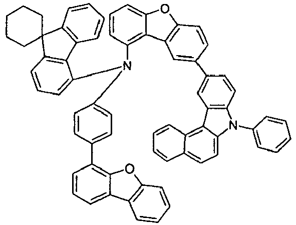 | 57% |
| j13 | 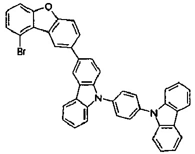 | 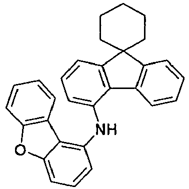 | 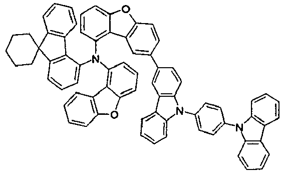 | 61% |
| j14 | 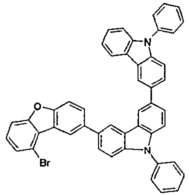 | 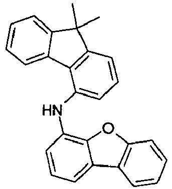 | 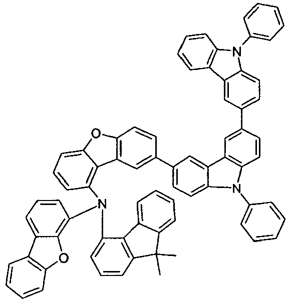 | 52% |
| j15 | 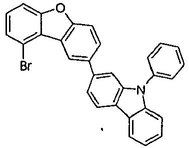 | 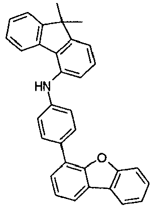 | 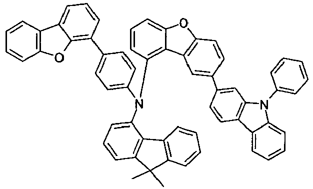 | 54% |
| j19 | 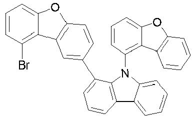 | 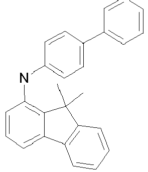 | 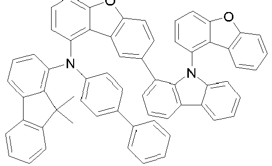 | 54% |
| j20 | 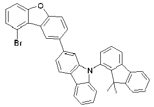 | 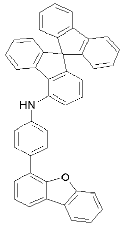 | 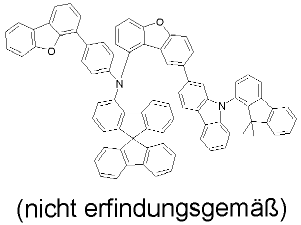 | 59% |
| j21 | 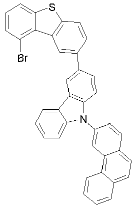 | 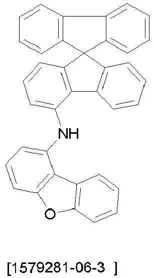 | 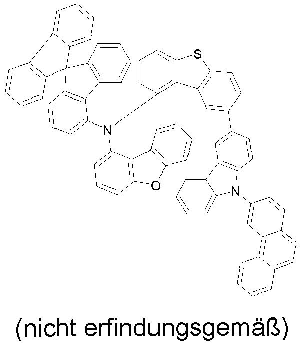 | 62% |
| j22 | 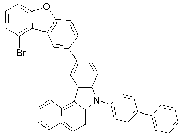 | 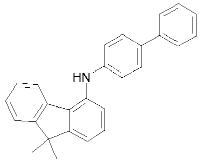 | 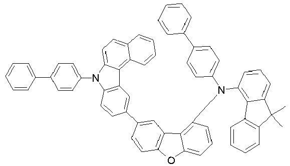 | 61% |
| j23 | 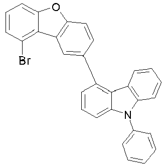 | 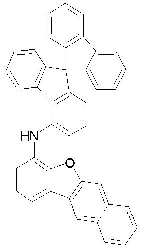 | 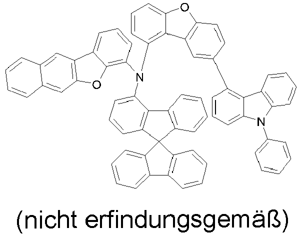 | 53% |
| j22 | 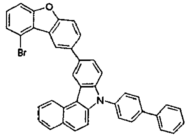 | 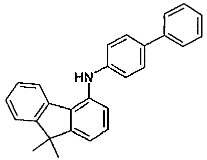 | 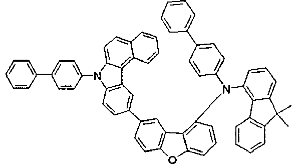 | 61% |
| j23 | 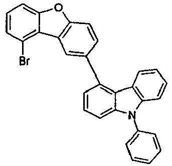 | 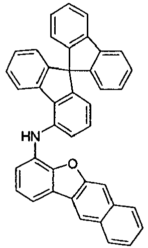 | 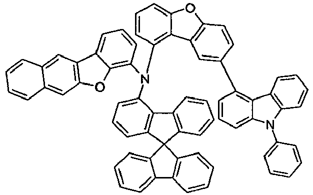 | 53% |
| j24 | 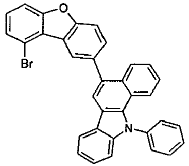 | 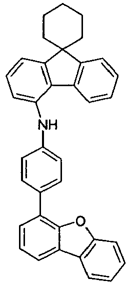 | 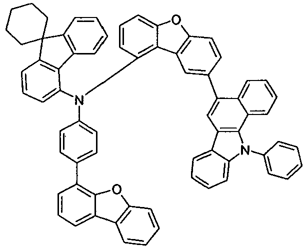 | 50% |
| j25 | 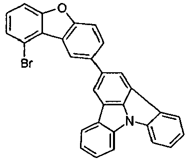 | 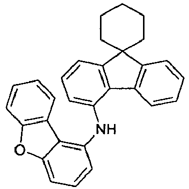 | 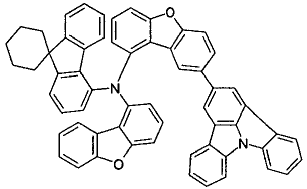 | 66% |
| j26 | 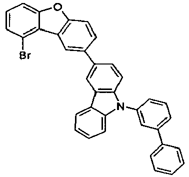 | 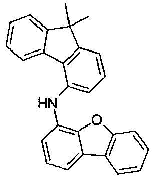 | 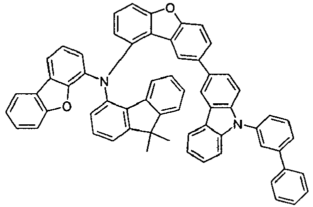 | 61% |
| j27 | 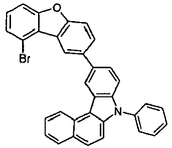 | 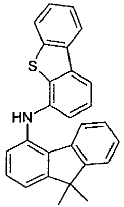 | 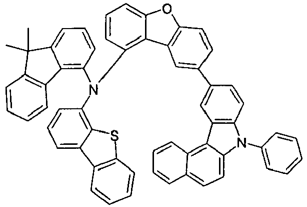 | 65% |
| j28 | 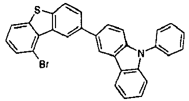 | 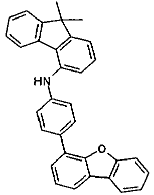 | 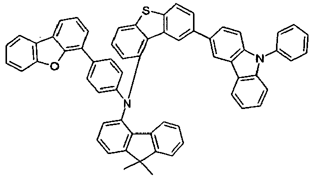 | 67% |
| j29 | 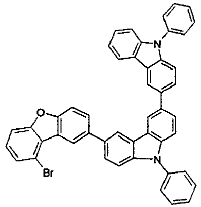 | 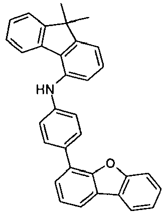 | 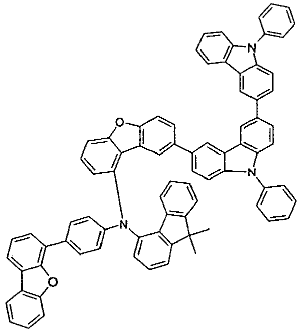 | 52% |
| j30 | 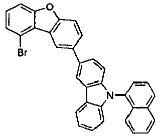 | 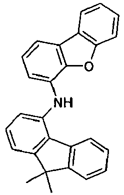 | 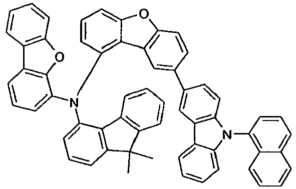 | 68% |
| j31 | 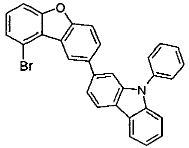 | 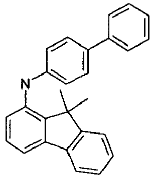 | 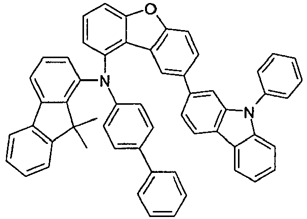 | 71% |

### Herstellung der OLEDs

In den folgenden Beispielen V1 bis E9 (siehe Tabellen 1 und 2) werden die Daten verschiedener OLEDs vorgestellt.

**Vorbehandlung für die Beispiele V1- E9:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf weiche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG1 (45%:45%:10%) 30nm bedeutet hierbei, dass das Material IC5 in einem Volumenanteil von 45%, IC3 in einem Anteil von 45% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Spannung und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt. Die Angabe U1000 in Tabelle 2 bezeichnet hierbei die Spannung, die für eine Leuchtdichte von 1000 cd/m² benötigt wird. EQE1000 bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L₁ absinkt. Eine Angabe von Lo;jo = 4000 cd/m² und L₁ = 70% in Tabelle X2 bedeutet, dass die in Spalte LD angegebene Lebensdauer der Zeit entspricht, nach der die Anfangsleuchtdichte von 4000 cd/m² auf 2800 cd/m² absinkt. Analog bedeutet Lo;jo = 20mA/cm², L₁= 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1 bis V8 sind Vergleichsbeispiele gemäß dem Stand der Technik, die Beispiele E1-E9 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz in der Emissionsschicht (EML) sowie auch in der Elektronenblockierschicht (EBL) phosphoreszierender OLEDs wesentliche Verbesserungen gegenüber dem Stand der Technik, vor allem bezüglich der Lebensdauer der OLED-Bauteile. Durch Einsatz der erfindungsgemäßen Verbindungen INV-1 bis INV-3 in Kombination mit IC5 und dem grünen Dotanden TEG1 lässt sich eine Erhöhung der Lebensdauer um mehr als 40% gegenüber dem Stand der Technik VG-1 bis VG-4 beobachten (Vergleich der Beispiele V1-V4 mit E1-E3). Durch Einsatz der erfindungsgemäßen Verbindungen INV-1 bis INV-3 in der EBL lässt sich eine Erhöhung der Lebensdauer um mehr als 25% gegenüber dem Stand der Technik VG-1 bis VG-4 beobachten (Vergleich der Beispiele V5-V8 mit E4-E6).

**Tabelle 1: Aufbau der OLEDs**

| Bsp. | HIL Dicke | IL Dicke | HTL Dicke | EBL Dicke | EML Dicke | HBL Dicke | ETL Dicke | EIL Dicke |
|---|---|---|---|---|---|---|---|---|
| V1 | SpA1 70nm | HATC N 5nm | SpMA1 70nm | --- | IC5:VG-1 :TEG1 (45%:45%:10%) 30nm | ST2 10nm | ST2:LiQ (50%:50%) 30nm | --- |
| V2 | SpA1 | HATC | SpMA1 | --- | IC5:VG-2:TEG1 | ST2 | ST2:LiQ | --- |
| | 70nm | N 5nm | 70nm | | (45%:45%:10%) 30nm | 10nm | (50%:50%) 30nm | |
| V3 | SpA1 70nm | HATC N 5nm | SpMA1 70nm | --- | IC5:VG-3:TEG1 (45%:45%:10%) 30nm | ST2 10nm | ST2:LiQ (50%:50%) 30nm | --- |
| V4 | SpA1 70nm | HATC N 5nm | SpMA1 70nm | --- | IC5:VG-4:TEG1 (45%:45%:10%) 30nm | ST2 10nm | ST2:LiQ (50%:50%) 30nm | --- |
| V5 | SpA1 70nm | HATC N 5nm | SpMA1 50nm | VG-1 20nm | IC1:TEG1 (90%:10%) 30nm | ST2 10nm | ST2:LiQ (50%:50%) 30nm | --- |
| V6 | SpA1 70nm | HATC N 5nm | SpMA1 70nm | VG-2 20nm | IC1:TEG1 (90%:10%) 30nm | ST2 10nm | ST2:LiQ (50%:50%) 30nm | --- |
| V7 | SpA1 70nm | HATC N 5nm | SpMA1 70nm | VG-3 20nm | IC1:TEG1 (90%:10%) 30nm | ST2 10nm | ST2:LiQ (50%:50%) 30nm | --- |
| V8 | SpA1 70nm | HATC N 5nm | SpMA1 70nm | VG-4 20nm | IC1:TEG1 (90%:10%) 30nm | ST2 10nm | ST2:LiQ (50%:50%) 30nm | --- |
| E1 | SpA1 70nm | HATC N 5nm | SpMA1 70nm | --- | IC5:INV-3:TEG1 (45%:45%:10%) 30nm | ST2 10nm | ST2:LiQ (50%:50%) 30nm | --- |
| E2 | SpA1 70nm | HATC N 5nm | SpMA1 70nm | --- | IC5:INV-1 :TEG1 (45%:45%:10%) 30nm | ST2 10nm | ST2:LiQ (50%:50%) 30nm | --- |
| E3 | SpA1 70nm | HATC N 5nm | SpMA1 70nm | --- | IC5:INV-2:TEG1 (45%:45%:10%) 30nm | ST2 10nm | ST2:LiQ (50%:50%) 30nm | --- |
| E4 | SpA1 70nm | HATC N 5nm | SpMA1 70nm | INV-3 20nm | IC1:TEG1 (90%:10%) 30nm | ST2 10nm | ST2:LiQ (50%:50%) 30nm | --- |
| E5 | SpA1 70nm | HATC N 5nm | SpMA1 70nm | INV-1 20nm | IC1:TEG1 (90%:10%) 30nm | ST2 10nm | ST2:LiQ (50%:50%) 30nm | --- |
| E6 | SpA1 | HATC | SpMA1 | INV-2 | IC1:TEG1 | ST2 | ST2:LiQ | --- |
| | 70nm | N 5nm | 70nm | 20nm | (90%:10%) 30nm | 10nm | (50%:50%) 30nm | |
| E7 | SpA1 70nm | HATC N 5nm | SpMA1 50nm | INV-3 20nm | IC5:IC3:TEG1 (45%:45%:10%) 30nm | ST2 10nm | ST2:LiQ (50%:50%) 30nm | --- |
| E8 | SpA1 70nm | HATC N 5nm | SpMA1 50nm | INV-1 20nm | IC5:IC3:TEG1 (45%:45%:10%) 30nm | ST2 10nm | ST2:LiQ (50%:50%) 30nm | --- |
| E9 | SpA1 70nm | HATC N 5nm | SpMA1 70nm | INV-2 20nm | IC5:IC3:TEG1 (45%:45%:10%) 30nm | ST2 10nm | ST2:LiQ (50%:50%) 30nm | --- |

**Tabelle 2: Daten der OLEDs:**

| Bsp. | U1000 (V) | EQE 1000 | L₀; j₀ | L₁ % | LD (h) |
|---|---|---|---|---|---|
| V1 | 3.1 | 15.3% | 20 mA/cm² | 80 | 200 |
| V2 | 3.2 | 15.4% | 20 mA/cm² | 80 | 180 |
| V3 | 3.2 | 15.2% | 20 mA/cm² | 80 | 150 |
| V4 | 3.3 | 15.5% | 20 mA/cm² | 80 | 140 |
| V5 | 3.1 | 16.8% | 20 mA/cm² | 80 | 105 |
| V6 | 3.2 | 15.7% | 20 mA/cm² | 80 | 100 |
| V7 | 3.1 | 15.6% | 20 mA/cm² | 80 | 90 |
| V8 | 3.2 | 15.1% | 20 mA/cm² | 80 | 85 |
| E1 | 3.2 | 16.1% | 20 mA/cm² | 80 | 280 |
| E2 | 3.3 | 16.3% | 20 mA/cm² | 80 | 260 |
| E3 | 3.3 | 16.2% | 20 mA/cm² | 80 | 240 |
| E4 | 3.2 | 17.2% | 20 mA/cm² | 80 | 135 |
| E5 | 3.3 | 17.0% | 20 mA/cm² | 80 | 130 |
| E6 | 3.2 | 17.5% | 20 mA/cm² | 80 | 120 |
| E7 | 3.2 | 17.0% | 20 mA/cm² | 80 | 255 |
| E8 | 3.3 | 16.8% | 20 mA/cm² | 80 | 250 |
| E9 | 3.4 | 17.3% | 20 mA/cm² | 80 | 240 |

**Tabelle 3: Verwendete Materialien:**

| | |
|---|---|
| 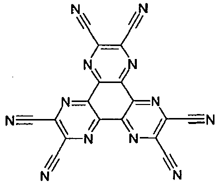 | 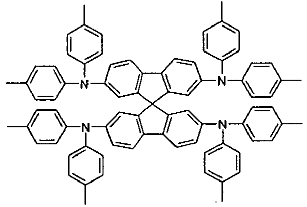 |
| HATCN | SpA1 |
| 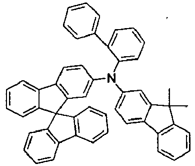 | 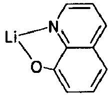 |
| SpMA1 | LiQ |
| 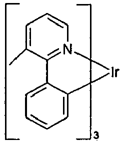 | 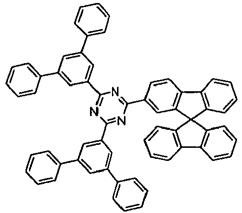 |
| TEG1 | ST2 |
| 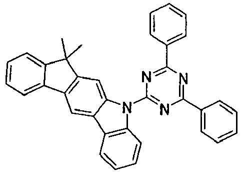 | 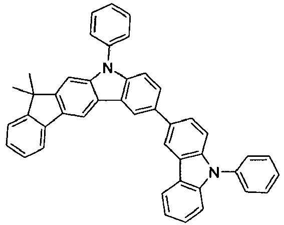 |
| IC1 | IC3 |
| 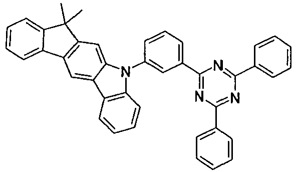 | 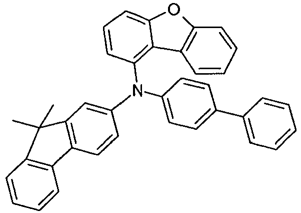 |
| IC5 | VG-1 |
| 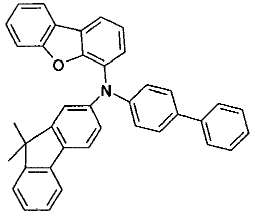 | 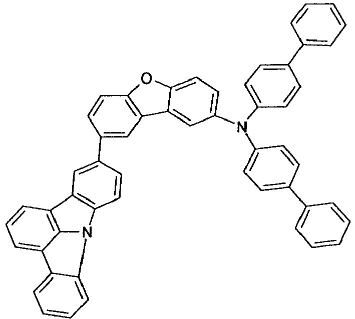 |
| VG-2 | VG-3 |
| 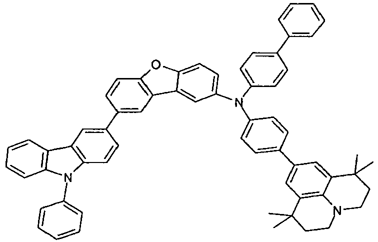 | |
| VG-4 | |
| 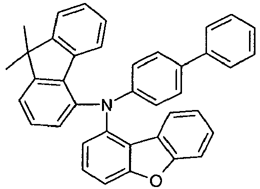 | 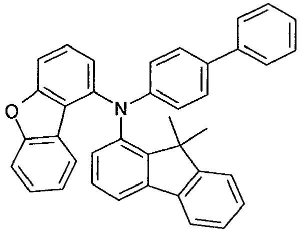 |
| INV-1 | INV-2 |
| 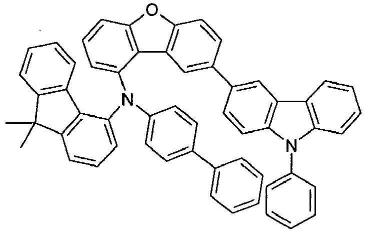 | |
| INV-3 | |

## Patentansprüche

1. Verbindung gemäß Formel (1), 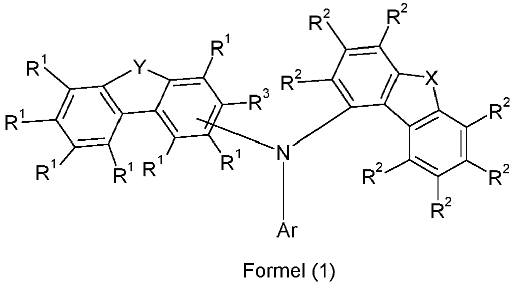 wobei für die verwendeten Symbole gilt:
X ist O oder S;
Y ist O, S oder CR₂;
Ar ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann, wobei Ar nur Arylgruppen oder Heteroarylgruppen mit bis zu 15 aromatischen Ringatomen und keine Carbazolylgruppen als Heteroarylgruppen, sowie keine 9,9'-Spirobifluorengruppen umfasst;
R ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R⁴ substituiert sein kann;
R¹ ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R¹ oder zwei Substituenten R¹ und R³, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
R² ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R², die an benachbarte Kohlenstoffatome gebunden sind, ein monocyclisches oder polycyclisches, aliphatisches Ringsystem bilden, das mit einem oder mehreren Resten R⁴ substituiert sein kann;
R³ ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R¹ und R³, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁴ substituiert sein kann; dabei können zwei Reste Ar¹, welche an dasselbe P-Atom oder B-Atom binden, auch durch eine Einfachbindung oder eine Brücke, ausgewählt aus N(R⁴), C(R⁴)₂, O oder S, miteinander verbrückt sein;
R⁴ ist bei jedem Auftreten gleich oder verschieden ausgewählt aus der Gruppe bestehend aus H, D, F, Cl, Br, I, CN, NO₂, N(R⁵)₂, C(=O)R⁵, einer geradkettigen Alkyl-, Alkoxy- oder Thioalkylgruppe mit 1 bis 20 C-Atomen oder einer verzweigten oder cyclischen Alkyl-, Alkoxyoder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nichtbenachbarte CH₂-Gruppen durch R⁵C=CR⁵, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei Substituenten R⁴, die an das gleiche Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R⁵ substituiert sein kann;
R⁵ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein mono- oder polycyclisches, aliphatisches Ringsystem bilden.
wobei einmal an Stelle von R¹ oder R³ das Stickstoffatom an das entsprechende Kohlenstoffatom gebunden ist, wobei Y im Falle von R³ nicht für CR₂ stehen darf.

2. Verbindung nach Anspruch 1, **dadurch gekennzeichnet, dass** X gleich O ist.

3. Verbindung nach einem oder mehreren der Ansprüche 1 oder 2, **dadurch gekennzeichnet, dass** Y gleich CR₂ ist.

4. Verbindung nach einem oder mehreren der Ansprüche 1 bis 3, **dadurch gekennzeichnet, dass** die Verbindung eine Verbindung der Formel (1-1) oder Formel (1-2) ist, 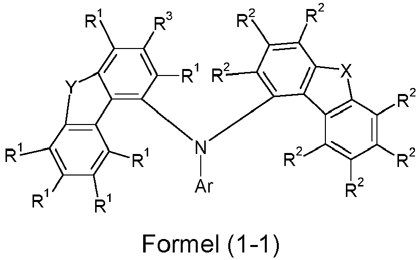 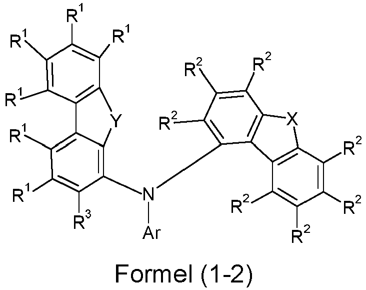 wobei die Symbole die in Anspruch 1 genannten Bedeutungen aufweisen.

5. Verbindung nach einem oder mehreren der Ansprüche 1 bis 4, **dadurch gekennzeichnet, dass** die Verbindung eine Verbindung der Formel 2 ist, 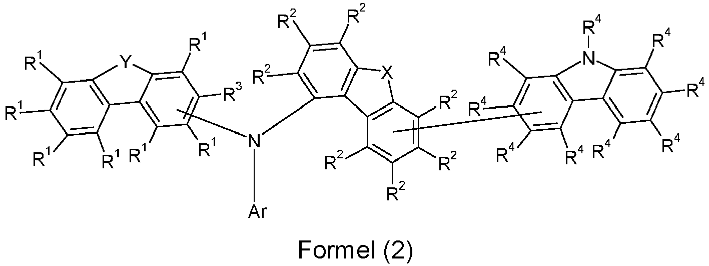 wobei die Symbole die in Anspruch 1 genannten Bedeutungen aufweisen, und wobei ein R² und ein an ein Kohlenstoffatom gebundener R⁴ durch die Einfachbindung ersetzt sind.

6. Verbindung nach einem oder mehreren der Ansprüche 1 bis 5, **dadurch gekennzeichnet, dass** die Verbindung eine Verbindung gemäß einer der Formeln (2-1) oder (2-2) ist, 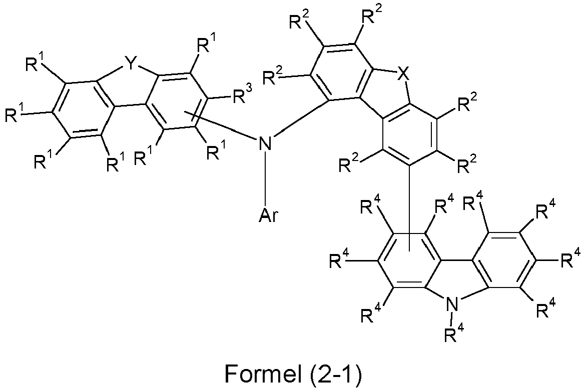 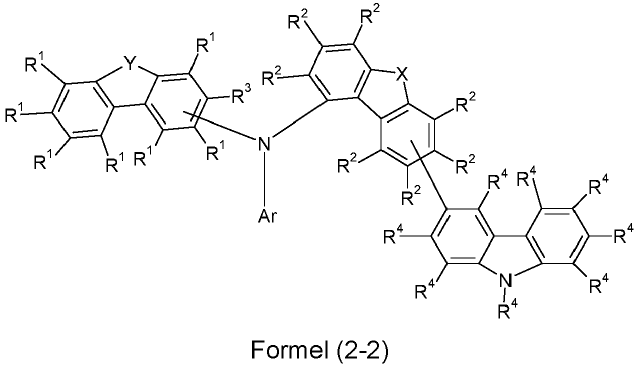 wobei die Symbole die in Anspruch 1 genannten Bedeutungen aufweisen, wobei in Formel (2-1) ein R² und in Formel (2-2) ein an ein Kohlenstoffatom gebundener R⁴ durch die Einfachbindung ersetzt sind.

7. Verbindung nach Anspruch 6, **dadurch gekennzeichnet, dass** in Formel (2-2) das durch die Einfachbindung ersetzte R² nicht an dem mit dem Stickstoffatom verbundenen Sechsring angeordnet ist.

8. Mischung enthaltend mindestens eine Verbindung nach einem oder mehreren der Ansprüche 1 bis 7 und mindestens eine weitere Verbindung und/oder mindestens ein Lösemittel.

9. Verwendung einer Verbindung nach einem oder mehreren der Ansprüche 1 bis 7 oder einer Mischung nach Anspruch 8 in einer elektronischen Vorrichtung.

10. Elektronische Vorrichtung enthaltend mindestens eine Verbindung nach einem oder mehreren der Ansprüche 1 bis 7 oder eine Mischung nach Anspruch 8.

11. Elektronische Vorrichtung nach Anspruch 10, **dadurch gekennzeichnet, dass** es sich um einer organische Elektrolumineszenzvorrichtung handelt.

12. Elektronische Vorrichtung nach Anspruch 11, **dadurch gekennzeichnet, dass** die Verbindung nach einem oder mehreren der Ansprüche 1 bis 7 oder die Mischung nach Anspruch 8 in einer emittierenden Schicht, bevorzugt in Kombination mit einem phosphoreszierenden Dotanden und gegebenenfalls einem oder mehreren weiteren Matrixmaterialien, oder in einer Lochtransportschicht oder in einer Elektronenblockierschicht eingesetzt wird.

## Claims

1. Compound of formula (1) 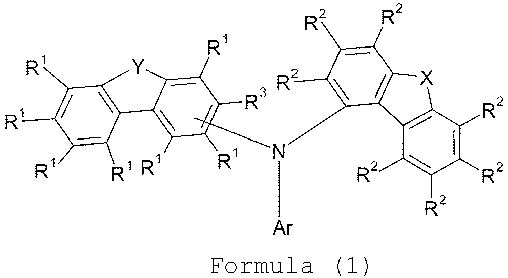 where the symbols used are as follows:
X is O or S;
Y is O, S or CR₂;
Ar is the same or different at each instance and is an aromatic or heteroaromatic ring system which has 5 to 40 aromatic ring atoms and may be substituted by one or more R radicals, where Ar comprises only aryl groups or heteroaryl groups having up to 15 aromatic ring atoms and no carbazolyl groups as heteroaryl groups, and no 9,9'-spirobifluorene groups;
R is the same or different at each instance and is selected from the group consisting of H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, a straightchain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O)(R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R¹ radicals, or an aralkyl or heteroaralkyl group which has 5 to 40 aromatic ring atoms and may be substituted by one or more R⁴ radicals; at the same time, it is optionally possible for two R substituents bonded to the same carbon atom or to adjacent carbon atoms to form a monocyclic or polycyclic aliphatic ring system which may be substituted by one or more R⁴ radicals;
R¹ is the same or different at each instance and is selected from the group consisting of H, D, F, Cl, Br, I, CN, NO₂, N(Ar¹)₂, N(R⁴)₂, C(=O)Ar¹, C(=O) R⁴, P(=O)(Ar¹)₂, P(Ar¹)₂, B(Ar¹)₂, a straightchain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O) (R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R¹ substituents or two R¹ and R³ substituents bonded to adjacent carbon atoms to form a monocyclic or polycyclic, aliphatic, aromatic or heteroaromatic ring system which may be substituted by one or more R⁴ radicals;
R² is the same or different at each instance and is selected from the group consisting of H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, a straightchain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O)(R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R² substituents bonded to adjacent carbon atoms to form a monocyclic or polycyclic, aliphatic ring system which may be substituted by one or more R⁴ radicals;
R³ is the same or different at each instance and is selected from the group consisting of H, D, F, Cl, Br, I, CN, NO₂, N(Ar¹)₂, N(R⁴)₂, C(=O)Ar¹, C(=O)R⁴, P(=O) (Ar¹)₂, P(Ar¹)₂, B(Ar¹)₂, a straightchain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O) (R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R¹ and R³ substituents bonded to adjacent carbon atoms to form a monocyclic or polycyclic, aliphatic, aromatic or heteroaromatic ring system which may be substituted by one or more R⁴ radicals;
Ar¹ is the same or different at each instance and is an aromatic or heteroaromatic ring system which has 5 to 30 aromatic ring atoms and may be substituted by one or more nonaromatic R⁴ radicals; at the same time, two Ar¹ radicals bonded to the same phosphorus atom or boron atom may also be bridged to one another by a single bond or a bridge selected from N(R⁴), C(R⁴)₂, O and S;
R⁴ is the same or different at each instance and is selected from the group consisting of H, D, F, Cl, Br, I, CN, NO₂, N(R⁵)₂, C(=O)R⁵, a straightchain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁵ radicals, where one or more nonadjacent CH₂ groups may be replaced by R⁵C=CR⁵, C=O, C=S, C=NR⁵, P(=O)(R⁵), SO, SO₂, NR⁵, O, S or CONR⁵ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁵ radicals, an aryloxy or heteroaryloxy group which has 5 to 40 aromatic ring atoms and may be substituted by one or more R⁵ radicals, or an aralkyl or heteroaralkyl group which has 5 to 40 aromatic ring atoms and may be substituted by one or more R⁵ radicals; at the same time, it is optionally possible for two R⁴ substituents bonded to the same carbon atom or adjacent carbon atoms to form a monocyclic or polycyclic, aliphatic, aromatic or heteroaromatic ring system which may be substituted by one or more R⁵ radicals;
R⁵ is the same or different at each instance and is selected from the group consisting of H, D, F, CN, an aliphatic hydrocarbyl radical having 1 to 20 carbon atoms, or an aromatic or heteroaromatic ring system having 5 to 30 aromatic ring atoms in which one or more hydrogen atoms may be replaced by D, F, Cl, Br, I or CN and which may be substituted by one or more alkyl groups each having 1 to 4 carbon atoms; at the same time, it is possible for two or more adjacent R⁵ substituents together to form a mono- or polycyclic, aliphatic ring system;
where, in one instance, the nitrogen atom is bonded to the corresponding carbon atom in place of R¹ or R³, where Y in the case of R³ cannot be CR₂.

2. Compound according to Claim 1, **characterized in that** X is O.

3. Compound according to one or more of Claims 1 and 2, **characterized in that** Y is CR₂.

4. Compound according to one or more of Claims 1 to 3, **characterized in that** the compound is a compound of the formula (1-1) or formula (1-2) 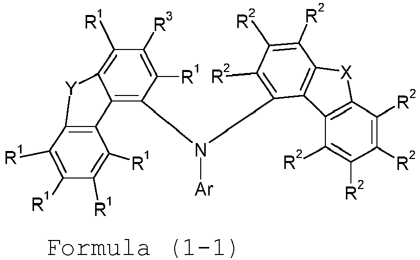 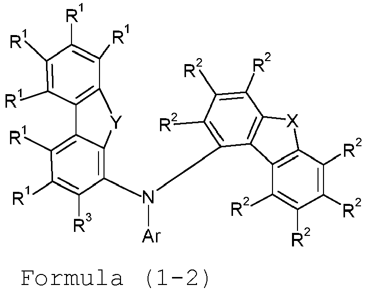 where the symbols have the definitions given in Claim 1.

5. Compound according to one or more of Claims 1 to 4, **characterized in that** the compound is a compound of the formula (2) 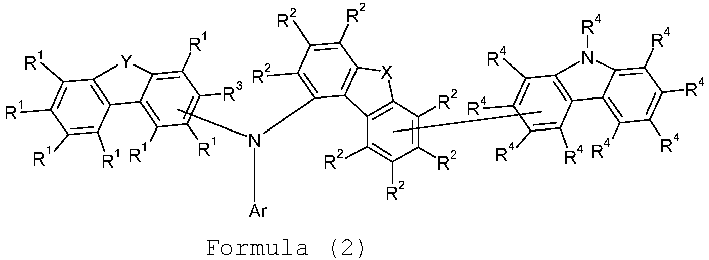 where the symbols have the definitions given in Claim 1, and where one R² and one R⁴ bonded to a carbon atom are replaced by the single bond.

6. Compound according to one or more of Claims 1 to 5, **characterized in that** the compound is a compound of one of the formulae (2-1) and (2-2) 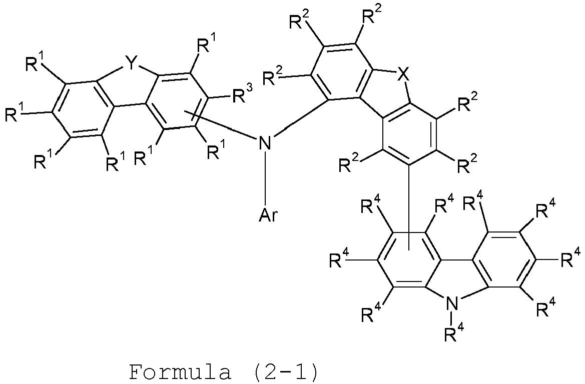 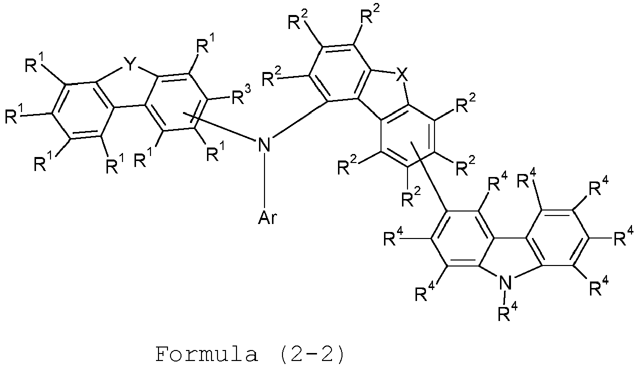 where the symbols have the definitions given in Claim 1, and where one R² in formula (2-1) and one R⁴ bonded to a carbon atom in formula (2-2) are replaced by the single bond.

7. Compound according to Claim 6, **characterized in that**, in formula (2-2), the R² replaced by the single bond is not arranged on the six-membered ring bonded to the nitrogen atom.

8. Mixture comprising at least one compound according to one or more of Claims 1 to 7 and at least one further compound and/or at least one solvent.

9. Use of a compound according to one or more of Claims 1 to 7 or a mixture according to Claim 8 in an electronic device.

10. Electronic device comprising at least one compound according to one or more of Claims 1 to 7 or a mixture according to Claim 8.

11. Electronic device according to Claim 10, **characterized in that** it is an organic electroluminescent device.

12. Electronic device according to Claim 11, **characterized in that** the compound according to one or more of Claims 1 to 7 or the mixture according to Claim 8 is used in an emitting layer, preferably in combination with a phosphorescent dopant and optionally one or more further matrix materials, or in a hole transport layer or in an electron blocker layer.

## Revendications

1. Composé selon la formule (1), 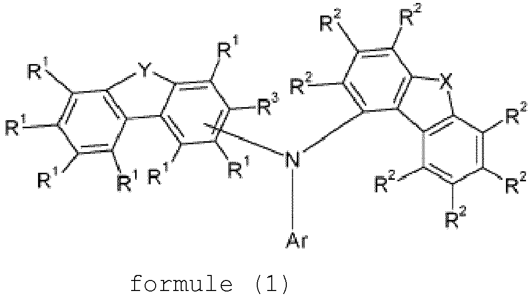 ce qui suit s'appliquant aux symboles utilisés :
X est O ou S ;
Y est O, S ou CR₂ ;
Ar, identique ou différent en chaque occurrence, est un système cyclique aromatique ou hétéroaromatique comportant 5 à 40 atomes de cycle aromatiques, qui peut être substitué par un ou plusieurs radicaux R, Ar ne comprenant que des groupes aryle ou des groupes hétéroaryle comportant jusqu'à 15 atomes de cycle aromatiques et pas de groupes carbazolyle en tant que groupes hétéroaryle, ainsi que pas de groupes 9,9'-spirobifluorène ;
R, identique ou différent en chaque occurrence, est choisi dans le groupe constitué par H, D, F, Cl, Br, I, CN, NO₂, N(Ar¹)₂, N(R⁴)₂, C(=O)Ar¹, C(=O)R⁴, P(=O) (Ar¹)₂, P(Ar¹)₂, B(Ar¹)₂, un groupe linéaire alkyle, alcoxy ou thioalkyle comportant 1 à 20 atome (s) de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁴, un ou plusieurs groupes CH₂ non voisins pouvant être remplacés par R⁴C=CR⁴, C=O, C=S, C=NR⁴, P(=O)(R⁴), SO, SO₂, NR⁴, O, S ou CONR⁴ et un ou plusieurs atomes de H pouvant être remplacés par D, F, Cl, Br, I, CN ou NO₂, un système cyclique aromatique ou hétéroaromatique comportant 5 à 40 atomes de cycle aromatiques, qui peut à chaque fois être substitué par un ou plusieurs radicaux R⁴, un groupe aryloxy ou hétéroaryloxy comportant 5 à 40 atomes de cycle aromatiques, qui peut être substitué par un ou plusieurs radicaux R¹, ou un groupe aralkyle ou hétéroaralkyle comportant 5 à 40 atomes de cycle aromatiques qui peut être substitué par un ou plusieurs radicaux R⁴ ; à cet égard deux substituants R, qui sont liés au même atome de carbone ou à des atomes de carbone voisins, peuvent éventuellement former un système cyclique aliphatique monocyclique ou polycyclique, qui peut être substitué par un ou plusieurs radicaux R⁴ ;
R¹, identique ou différent en chaque occurrence, est choisi dans le groupe constitué par H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, un groupe linéaire alkyle, alcoxy ou thioalkyle comportant 1 à 20 atome (s) de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁴, un ou plusieurs groupes CH₂ non voisins pouvant être remplacés par R⁴C=CR⁴, C=O, C=S, C=NR⁴, P(=O)(R⁴), SO, SO₂, NR⁴, O, S ou CONR⁴ et un ou plusieurs atomes de H pouvant être remplacés par D, F, Cl, Br, I, CN ou NO₂, un système cyclique aromatique comportant 5 à 40 atomes de cycle aromatiques, qui peut à chaque fois être substitué par un ou plusieurs radicaux R⁴, un groupe aryloxy ou hétéroaryloxy comportant 5 à 40 atomes de cycle aromatiques, qui peut être substitué par un ou plusieurs radicaux R⁴, ou un groupe aralkyle ou hétéroaralkyle comportant 5 à 40 atomes de cycle aromatiques qui peut être substitué par un ou plusieurs radicaux R⁴ ; à cet égard deux substituants R¹ ou deux substituants R¹ et R³, qui sont liés à des atomes de carbone voisins, peuvent éventuellement former un système cyclique aliphatique, aromatique ou hétéroaromatique, monocyclique ou polycyclique, qui peut être substitué par un ou plusieurs radicaux R⁴ ;
R², identique ou différent en chaque occurrence, est choisi dans le groupe constitué par H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, un groupe linéaire alkyle, alcoxy ou thioalkyle comportant 1 à 20 atome (s) de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁴, un ou plusieurs groupes CH₂ non voisins pouvant être remplacés par R⁴C=CR⁴, C=O, C=S, C=NR⁴, P(=O)(R⁴), SO, SO₂, NR⁴, O, S ou CONR⁴ et un ou plusieurs atomes de H pouvant être remplacés par D, F, Cl, Br, I, CN ou NO₂, un système cyclique aromatique ou hétéroaromatique comportant 5 à 40 atomes de cycle aromatiques, qui peut à chaque fois être substitué par un ou plusieurs radicaux R⁴, un groupe aryloxy ou hétéroaryloxy comportant 5 à 40 atomes de cycle aromatiques, qui peut être substitué par un ou plusieurs radicaux R⁴, ou un groupe aralkyle ou hétéroaralkyle comportant 5 à 40 atomes de cycle aromatiques qui peut être substitué par un ou plusieurs radicaux R⁴ ; à cet égard deux substituants R², qui sont liés à des atomes de carbone voisins, peuvent éventuellement former un système cyclique aliphatique monocyclique ou polycyclique, qui peut être substitué par un ou plusieurs radicaux R⁴ ;
R³, identique ou différent en chaque occurrence, est choisi dans le groupe constitué par H, D, F, Cl, Br, I, CN, NO₂, N(Ar¹)₂, N(R⁴)₂, C(=O)Ar¹, C(=O)R⁴, P(=O) (Ar¹)₂, P(Ar¹)₂, B(Ar¹)₂, un groupe linéaire alkyle, alcoxy ou thioalkyle comportant 1 à 20 atome (s) de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁴, un ou plusieurs groupes CH₂ non voisins pouvant être remplacés par R⁴C=CR⁴, C=O, C=S, C=NR⁴, P(=O)(R⁴), SO, SO₂, NR⁴, O, S ou CONR⁴ et un ou plusieurs atomes de H pouvant être remplacés par D, F, Cl, Br, I, CN ou NO₂, un système cyclique aromatique ou hétéroaromatique comportant 5 à 40 atomes de cycle aromatiques, qui peut à chaque fois être substitué par un ou plusieurs radicaux R⁴, un groupe aryloxy ou hétéroaryloxy comportant 5 à 40 atomes de cycle aromatiques, qui peut être substitué par un ou plusieurs radicaux R⁴, ou un groupe aralkyle ou hétéroaralkyle comportant 5 à 40 atomes de cycle aromatiques qui peut être substitué par un ou plusieurs radicaux R⁴ ; à cet égard deux substituants R¹ et R³, qui sont liés à des atomes de carbone voisins, peuvent éventuellement former un système cyclique aliphatique, aromatique ou hétéroaromatique, monocyclique ou polycyclique, qui peut être substitué par un ou plusieurs radicaux R⁴ ;
Ar¹, identique ou différent en chaque occurrence, est un système cyclique aromatique ou hétéroaromatique comportant 5 à 30 atomes de cycle aromatiques qui peut être substitué par un ou plusieurs radicaux non aromatiques R⁴ ; à cet égard, deux radicaux Ar¹ qui sont liés au même atome de P ou au même atome de B, peuvent être pontés l'un avec l'autre par l'intermédiaire d'une liaison simple ou d'un pont, choisi parmi N(R⁴), C(R⁴)₂, O et S ;
R⁴, identique ou différent en chaque occurrence, est choisi dans le groupe constitué par H, D, F, Cl, Br, I, CN, NO₂, N(R⁵)₂, C(=O)R⁵, un groupe linéaire alkyle, alcoxy ou thioalkyle comportant 1 à 20 atome (s) de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁵, un ou plusieurs groupes CH₂ non voisins pouvant être remplacés par R⁵C=CR⁵, C=O, C=S, C=NR⁵, P(=O)(R⁵), SO, SO₂, NR⁵, O, S ou CONR⁵ et un ou plusieurs atomes de H pouvant être remplacés par D, F, Cl, Br, I, CN ou NO₂, un système cyclique aromatique ou hétéroaromatique comportant 5 à 40 atomes de cycle aromatiques, qui peut être à chaque fois substitué par un ou plusieurs radicaux R⁵, un groupe aryloxy ou hétéroaryloxy comportant 5 à 40 atomes de cycle aromatiques qui peut être substitué par un ou plusieurs radicaux R⁵, ou un groupe aralkyle ou hétéroaralkyle comportant 5 à 40 atomes de cycle aromatiques, qui peut être substitué par un ou plusieurs radicaux R⁵ ; à cet égard, deux substituants R⁴ qui sont liés au même atome de carbone ou à des atomes de carbone voisins, peuvent éventuellement former un système cyclique aliphatique, aromatique ou hétéroaromatique monocyclique ou polycyclique, qui peut être substitué par un ou plusieurs radicaux R⁵ ;
R⁵, identique ou différent en chaque occurrence, est choisi dans le groupe constitué par H, D, F, CN, un radical hydrocarboné aliphatique comportant 1 à 20 atome (s) de C ou un système cyclique aromatique ou hétéroaromatique comportant 5 à 30 atomes de cycle aromatiques, dans lequel un ou plusieurs atomes de H peuvent être remplacés par D, F, Cl, Br, I ou CN et qui peut être substitué par un ou plusieurs groupes alkyle comportant à chaque fois 1 à 4 atome(s) de carbone ; à cet égard, deux substituants R⁵ voisins ou plus peuvent former ensemble un système cyclique aliphatique monocyclique ou polycyclique ;
l'atome d'azote étant, une fois à la place de R¹ ou de R³, lié à l'atome de carbone correspondant, Y, dans le cas de R³, ne pouvant pas représenter CR₂.

2. Composé selon la revendication 1, **caractérisé en ce que** X est égal à O.

3. Composé selon l'une ou plusieurs des revendications 1 et 2, **caractérisé en ce que** Y est égal à CR₂.

4. Composé selon l'une ou plusieurs des revendications 1 à 3, **caractérisé en ce que** le composé est un composé de formule (1-1) ou de formule (1-2), 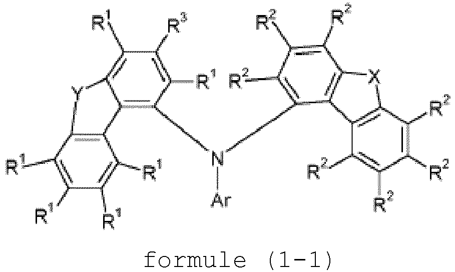 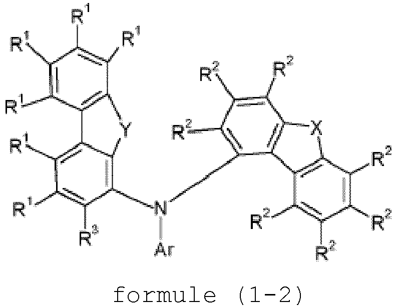 les symboles présentant les significations mentionnées dans la revendication 1.

5. Composé selon l'une ou plusieurs des revendications 1 à 4, **caractérisé en ce que** le composé est un composé de formule (2), 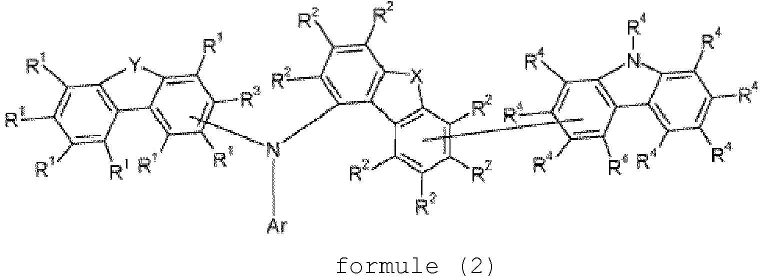 les symboles présentant les significations mentionnées dans la revendication 1, et un R² et un R⁴ liés à un atome de carbone sont remplacés par la simple liaison.

6. Composé selon l'une ou plusieurs des revendications 1 à 5, **caractérisé en ce que** le composé est un composé selon l'une des formules (2-1) et (2-2), 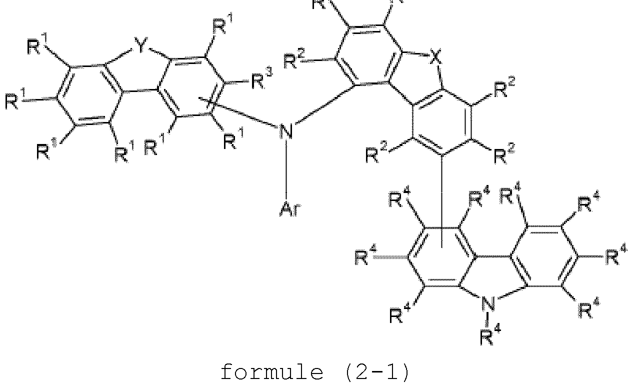 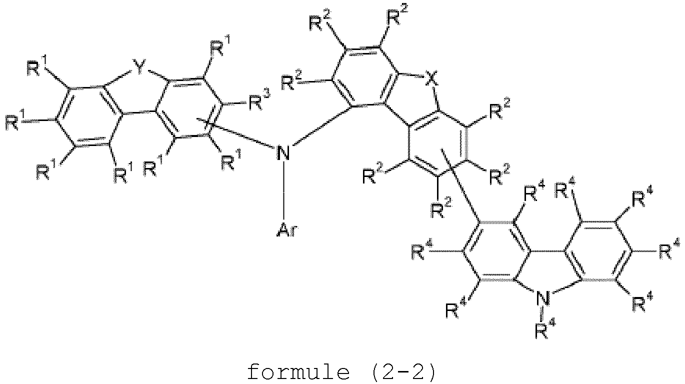 les symboles présentant les significations mentionnées dans la revendication 1, où dans la formule (2-1) un R² et dans la formule (2-2) un R⁴ lié à un atome de carbone sont remplacés par la simple liaison.

7. Composé selon la revendication 6, **caractérisé en ce que** dans la formule (2-2), le R² remplacé par la simple liaison n'est pas placé au niveau du cycle à six chaînons lié à l'atome d'azote.

8. Mélange contenant au moins un composé selon l'une ou plusieurs des revendications 1 à 7 et au moins un composé supplémentaire et/ou au moins un solvant.

9. Utilisation d'un composé selon l'une ou plusieurs des revendications 1 à 7 ou d'un mélange selon la revendication 8 dans un dispositif électronique.

10. Dispositif électronique contenant au moins un composé selon l'une ou plusieurs des revendications 1 à 7 ou un mélange selon la revendication 8.

11. Dispositif électronique selon la revendication 10, **caractérisé en ce que** c'est un dispositif organique d'électroluminescence.

12. Dispositif électronique selon la revendication 11, **caractérisé en ce que** le composé selon l'une ou plusieurs des revendications 1 à 7 ou le mélange selon la revendication 8 est utilisé dans une couche émettrice, préférablement en combinaison avec un dopant phosphorescent et éventuellement un ou plusieurs matériaux de matrice supplémentaires, ou dans une couche de transport de trous ou dans une couche de blocage d'électrons.
